Europäisches Patentamt

European Patent Office

Office européen des brevets

(19)

(11) Publication number : **0 436 467 A2**

(12)

# EUROPEAN PATENT APPLICATION

(21) Application number : 90811009.1

(22) Date of filing : 20.12.90

(51) Int. Cl.⁵ : **C12N 15/29, C12N 15/82, A01H 5/00, C12Q 1/68**

(71) Applicant : **CIBA-GEIGY AG**
**Klybeckstrasse 141**
**CH-4002 Basel (CH)**

(72) Inventor : **Nasrallah, June B.**
**109 Brook Drive**
**Ithaca, NY 14850 (US)**
Inventor : **Nasrallah, Mikhail E.**
**109 Brook Drive**
**Ithaca, NY 14850 (US)**
Inventor : **Miflin, Benjamin J.**
**Maiengasse 12**
**CH-4056 Basle (CH)**
Inventor : **Thorsness, Mary K.**
**160 Glies Street**
**Ithaca, NY 14850 (US)**

the applicant has filed a statement in accordance with Rule 28 (4) EPC (issue of a sample only to an expert). Accession number(s) of the deposit(s): ATCC 67113

A request for correction by renumbering the claims and deleting claim n°18 has been filed pursuant to Rule 88 EPC. A decision on the request will be taken during the proceedings before the Examining Division (Guidelines for Examination in the EPO, A-V, 2.2).

(30) Priority : **29.12.89 US 459069**
**04.06.90 US 532907**

(43) Date of publication of application :
**10.07.91 Bulletin 91/28**

(84) Designated Contracting States :
**AT BE CH DE DK ES FR GB GR IT LI LU NL SE**

(54) Expression of S-locus specific glycoprotein gene in transgenic plants.

(57) The present invention describes the introduction of a S-locus specific glycoprotein gene isolated from one plant strain into another plant strain that does not ordinarily synthesize S-locus specific glycoprotein.

EP 0 436 467 A2

Jouve, 18, rue Saint-Denis, 75001 PARIS

| DAYS BEFORE ANTHESIS | MICROSPORE AND POLLEN STAGES | GUS ACTIVITY IN | |
|---|---|---|---|
| | | ANTHER | STIGMA |
| 9 | PRE- MEIOSIS | | |
| 8 | | | |
| 7 | TETRAD | | |
| 6 | UNI- NUCLEATE | | |
| 5 | | | |
| 4 | BI- NUCLEATE | | |
| 3 | | | |
| 2 | TRI- NUCLEATE | | |
| 1 | | | |
| 0 | | | |

FIG. 4

# EXPRESSION OF S-LOCUS SPECIFIC GLYCOPROTEIN GENE IN TRANSGENIC PLANTS

The present invention relates to self-incompatible plants comprising a structural gene encoding a S-locus specific glycoprotein, to recombinant transfer vectors comprising the said structural gene and to a method of imparing self-incompatibility to recipient plants by use of the said recombinant transfer vectors.

The present invention further relates to tissue specific promoter sequences that are capable of directing the expression of an associated structural gene, preferably of a S-locus specific glycoprotein, to a desired plant tissue, preferably to the reproductive tissue of a plant, and to a method of obtaining tissue specific expression of a gene such as, for example, a S-locus specific glycoprotein in a plant, especially in the reproductive tissue of a plant.

Also comprised by the present invention is a method for hybrid production of self-incompatible plants.

Plant science has recognized for many years that hybridization of closely related plants often results in the production of an offspring ($F_1$) generation having a combination of desirable traits which previously were possessed separately by the parent plants. Also recognized is that hybrid plants of various crop species commonly have possessed vigor or heterosis which has contributed significantly to the species'crop yield. Accordingly, many hybrid crosses have been of considerable economic importance.

Since plants selected for hybridization studies are commonly capable of undergoing both self-pollination (i.e., the plants are self compatible) and crosspollination, the desired hybrid crosses have been difficult to achieve on a reliable basis while operating on a commercial basis. In order to achieve hybrid crosses on a commercial scale, it is necessary to have controlled crosspollination in the substantial absence of self-pollination (i.e., plants that are self-incompatible).

In more than 3,000 species representing diverse angiosperm families, self-fertilization is blocked by a genetically controlled mechanism termed "self-incompatibility". This phenomenon facilitates out-crossing by inhibiting growth of self pollen in pistil tissue. Following self-pollination, interaction between the stigma or style and pollen elicits a defined morphological response preventing normal pollen tube growth. Fertilization is thus prevented as the pollen tubes are unable to penetrate the full lengt of the stigma and style and the gamete nuclei of the pollen are unable to reach the female gametophyte tissue. The specificity of this interaction is determined by one or more genetic loci, and requires the identity of alleles carried by the male and female parents.

The control of incompatibility is attributed to a single genetic locus with multiple alleles. As with other allelomorphs, any two alleles may be carried by a diploid plant, and incompatibility occurs if the alleles expressed in pollen and pistil are identical.

Incompatibility systems are generally divided into two major groups : the gametophytic system in which the phenotype of a pollen grain is determined by the haploid pollen genotype is the first, and the sporophytic system in which the pollen phenotype is determined by the genotype of the parental plant is the second. In the sporophytic system of incompatibility, exemplified by the genus *Brassica*, and other crucifers belonging to the family *Brassicaceae* (which includes such plants as broccoli, cabbage, kale, mustards, oil seed crops and radish), the self-incompatible inhibition is localized at the stigma surface and occurs within minutes by the failure of self-pollen to germinate and/or invade the papillar cells on the outer surface of the stigma. Thus, self-fertilization is prevented.

In this *Brassicaceae* family, self-incompatibility is is under the control of a single multi-allelic locus (numerous alleles have been identified in natural populations), called the S-locus, and the phenotype of pollen is determined by the diploid genotpye of the sporophyte [see A.J. Bateman (1955)]. While the mechanism of pollen recognition in plants is not understood, the observation that a self-incompatibility response occurs when the same S-allele is active in pollen and stigma suggests that pollen inhibition is based on the expression of a single S-locus gene in the male and female structures of the flower. Furthermore, the sporophytic control of pollen phenotype has been postulated to result from the expression of this S-locus gene in the tapetum, the layer of sporophytic cells that lines the anther and whose function is to nourish the developing pollen grains [see J. Heslop-Harrison (1968) and J. Heslop-Harrison (1975)]. Based largely on ultrastructural observations, this layer of cells is thought to act as nurse cells for developing microspores, and to synthesize and release materials that are incorporated into the outer coat of pollen grains, the exine, just prior to and during tapetal dissolution late in pollen development.

A gene involved in the specific recognition of self pollen is also expected to be sporophytically expressed in the host's anthers during pollen development. While blot analysis of anther RNA has identified a low-abundance transcript with homology to the S-locus glycoprotein gene [see J.B. Nasrallah *et al.* (1989)] these studies could not distinguish between expression of the S-locus glycoprotein related genes found in the *Brassica* genome [see K.G. Dwyer *et al.* (1989)]. The putative S-locus genetic protein of pollen has and continues to remained elusive.

Perhaps the most experimentally tractable route towards a molecular analysis of the genetic control of incompatibility is suggested by the detection of antigens specific to various S-locus alleles in stigma homogenates from different *Brassica* strains. These antigens have been shown to correspond to glycoproteins which may be resolved in various electrophoretic systems. Several lines of evidence suggest that these glycoproteins play an important role in incompatibility : (1) the mobilities of these molecules vary in stigma extracts derived from *Brassica* strains with different S-locus alleles ; (2) these molecules are found exclusively in the stigma (specifically in the surface papillar cells on the stigma surface), the site of the initial contact between the pollen and stigma ; (3) an increased rate of synthesis of these S-locus specific glycoproteins in the developing stigma correlates well with the onset of the incompatibility reaction in the stigma ; and (4) in the stigma of self-incompatible lines of *B. oleracea* (kale), *B. campestris*, and the closely related *Raphanus sativus* (radish) glycoproteins have been identified which cosegregate with their corresponding S-alleles in the $F_1$ and $F_2$ generations, indicating that the gene responsible for this polymorphism is at the S-locus.

These glycoproteins have been designated S-locus specific glycoprotein ("SLSG"). Each S genotype has its own unique glycoprotein, and to date more than twenty different SLSG have been correlated with S-alleles. Furthermore, SLSG encoded by different S alleles exhibit extensive polymorphism and are distinguishable from one another on the basis of their characteristic charge, molecular weight and antigenic properties. In addition, SLSG accumulate during stigma development and are synthesized at a maximal rate coincident with the acquisition by the developing stigma of the ability to reject self pollen.

The cell-specific expression of these genes in the stigma has been well characterized. S-locus gene transcripts were localized by *in situ* hybridization to the papillar cells of the stigma surface [see J.B. Nasrallah *et al.* (1988)], and S-locus gene glycoprotein products were shown by ultrastructural immunocytochemistry to accumulate in the papillar cell walls [see M.K. Kandasamy *et al.* (1989)]. These results, combined with the genetic linkage of S-locus glycoprotein genes to the S-locus, have led to one aspect of the present invention, that is, that these genes encode the stigmatic determinants of self-incompatibility.

Finally analysis of *Brassica* genomic DNA has revealed extensive restriction fragment length polymorphisms which cosegregate with the corresponding S alleles in genetic crosses. These developmental, cytological and genetic data strongly suggest that SLSG are the products of the S-locus and that they act as recognition substances in the self-incompatibility reaction.

Accordingly, it has been one of the main objectives of the present invention to develop a method of imparting self-incompatibility to recipient plants by transforming self-incompatibility genes into appropriate plant strains.

This and other objectives could be surprisingly met within the scope of the present invention.

Isolation of a cDNA clone encoding an S-locus specific glycoprotein is not only of scientific but also of commercial interest because such a clone would allow for the identification of self-incompatibility genes and the introduction of these genes into appropriate plant strains by transformation. Additionally, it would be possible for transformed strains carrying the S-alleles to be interplanted with strains carrying different S-alleles. The seed harvested from such an interplanting would be entirely hybrid. Hybrids are already known to be better suited for breeding disease resistant varieties, produce higher yields, and have better adaptability to extreme environmental growth conditions. Another advantage, in ornamental crops for example, is that self-incompatible varieties have a longer shelf life and flower over a longer season.

When used as a probe on blots of genomic DNA prepared from *Brassica, Raphanus, Arabidopsis* and other crucifers, SLSG-encoding cDNAs hybridize with several restriction fragments indicating that SLSG-related sequences occur as a multigene family in crucifers.

Members of this multigene family can be grouped into two classes. One class comprises genes linked at the S-locus including 1) the SLSG structural gene, referred to as the SLG gene, and 2) additional related genes. Another class comprises genes related to SLG genes but unlinked to the S-locus, and are designated "S-locus related" (SLR) genes. All these genes also exhibit considerable (60-80 %) homology at the amino acid level.

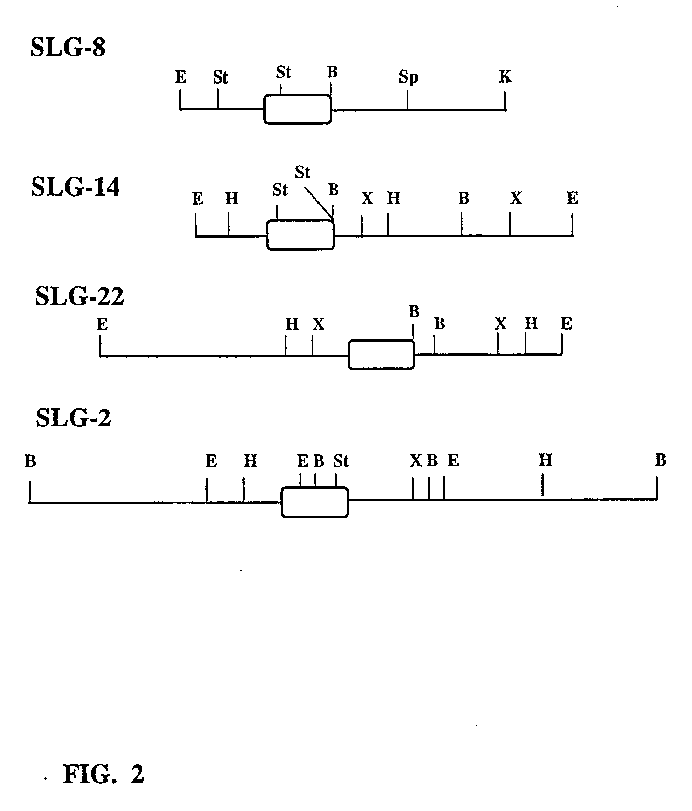

Within any one S-allele homozygote, the SLG gene can be identified from among the other related gene copies by hybridization to a gene-specific probe derived from the 3'untranslated end of SLSG-encoding cDNA. By using this 3'-end gene specific probe, the SLG gene can be isolated from the S13, S14 and S22 homozygous lines of *Brassica oleracea,* and from the S8 homozygous line of *B. campestris*. The cloned genes are referred to as SLG-13, SLG-14, SLG-22 and SLG-8 respectively. The restriction maps of these genes and flanking regions are shown in Figure 2.

The determination of a substantially pure DNA base sequence coding for an S-locus specific glycoprotein from a self-incompatible strain of *Brassica,* the construction of novel recombinant DNA transfer vectors containing the cloned DNA, and transformed microorganisms transformed by the recombinant DNA transfer verctors have been already described in EP application 212,385.

SLR genes have been isolated from a number of different S-allele homozygous genotypes. To date, two SLR genes have been shown to be expressed. These genes are referred to as SLR1 and SLR2. Both genes are expressed in the papillar cells of the stigma surface and have the same pattern of expression during flower development as the SLG genes. They therefore have common regulatory sequences that direct their cell-type specific expression.

The SLR1 gene can be isolated from the S2, S6, S13, S15, S22 homozygotes of *B. oleracea* and from the Yellow Sarson variety of *B. campestris* [except for S15, these have been reported in Lalonde *et al* (1989)].

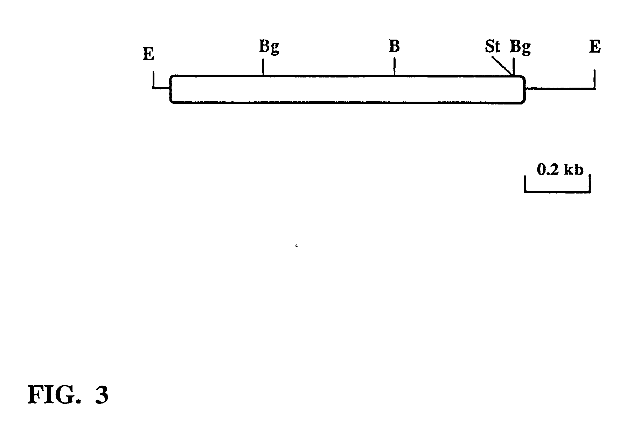

The SLR2 gene can be isolated from the S2, S5, S6 and S15 homozygous genotypes of *B. oleracea*. A map of SLR2-cDNA isolated from the S2 and S15 genotypes is shown in Figure 3.

With regard to the present invention, it is demonstrated that the introduction of SLG gene isolated and molecularly cloned from one plant strain may be introduced into another plant strain not known to synthesize SLSG. This is specifically demonstrated for two members of the family *Brassicaceae* wherein a SLG gene isolated from the S13 and S8 alleles of *Brassica oleracea* homozygote is introduced into a self-compatible *Brassica napus* strain not known to synthesize SLSG. This is also demonstrated using molecularly cloned SLSG genes in other members of the *Brassicacea*, specifically *Arabidopsis thaliana*, (specifically SLG-8, SLG-13, SLG-14, and SLG-22 using a vector similar to that described in Example 1) and in plants not within this family as, for example, in *Nicotiana alata* and *Nicotiana tabacum* (specifically SLG-8, SLG-13, SLG-14, and SLG-22). In fact, as *Nicotiana* belongs to the gametophytic system of incompatibility, this introduction of a SLG gene demonstrates that the cloned gene may also be transferred successfully between plants of differing incompatibility systems.

The transformation of a molecularly cloned gene is important for a number of reasons, including the commercial hybrid production of seed. While hybrid seed production by the transformation of a molecularly cloned gene is similar to published (due to the feasibility of *Brassica napus* and *Brassica campestris* crosses, S-alleles have been introgessed into *Brassica napus* from the parental *Brassica campestris* species ; reported by G.R. Mackay (1977) introgessed S-allele techniques, there are a number of advantages that are only found in the techniques described for the present invention. For example, using molecularly cloned gene transformation, only the incompatibility S gene and the phenotype it confers are acquired by the recipient lines - something not possible with published introgessed S-allele techniques which are more time consuming and normally include the introduction of unwanted genetic material by "linkage drag". Furthermore, using molecularly cloned transformation, the S gene for incompatibility may be linked with a gene for herbicide tolerance, such as those genes yielding a tolerance (or resistance) to hygromycin, kanamycin, phosphinotricin, asulam, sulfonyl ureas, glyphosate, bromoxymil, or imidazolinones, for co-transformation of the two genes into a single female plant.

When the parent female plant is crossed with a male having desired characteristics not found in the female, such as would occur in the commercial production of hybrid seed by interplanting, the field, after pollination but prior to harvest of the seed, may be treated with a herbicide to destroy all herbicide sensitive male plants. Since the transformed female plant is herbicide tolerant, only the female plant will survive herbicide treatment, and only hybrid seeds from the female plant (which will carry genes from the interplanted male) will mature and be harvested.

Thus, one of the main objectives of the present invention was the development of a method of imparting self-incompatibility to recipient plants by transforming self-incompatibility into appropriate plant strains.

In particular, the present invention relates to a method of imparting self-incompatibility to recipient plants comprising the following steps :

(a) isolating a structural gene which encodes for the production of an S-locus specific glycoprotein from a self-incompatible donor plant, preferably a self-incompatible strain of *Brassica* ; and

(b) integrating said structural gene into the genome of a recipient plant that does not ordinarily produce said S-locus specific glycoprotein, wherein said recipient plant is self-incompatible.

Preferred is a method, wherein the structural gene is selected from the group consisting of the SLG-13, S-14, SLG-22 genes of *Brassica oleracea* and the SLG-8 gene of *Brassica campestris*. Especially preferred is a method wherein the structural gene comprises a DNA sequence as given in sequence listing 1.

Also comprised by the present invention is a method of obtaining tissue specific expression of a gene in a plant comprising :

(a) isolating a tissue specific plant promoter from the reproductive tissue of a donor plant ;

(b) linking the tissue specific plant promoter with a structural gene, preferably a structural gene encoding for a S-locus specific glycoprotein, such that the structural gene is specifically expressed only in the reproductive tissue, preferably in the pistil and/or anther of a recipient plant.

Preferred is a method for obtaining a self-incompatible plant from a species which is not normally self-incompatible by tissue specific expression of a S-locus specific glycoprotein, which method comprises the following steps :

(a) isolating a tissue specific plant promoter from the reproductive tissue of a donor plant ;

(b) operably linking the tissue specific plant promoter with a DNA sequence comprising a region of a S-locus specific gene of sufficient length to express a S-locus specific glycoprotein substantially similar to that expressed in *Brassica* sp. ;

(c) optionally fusing a reporter gene to the resulting linked promoter-gene ;

(d) inserting the resulting chimeric gene into the genome of a recipient plant which is not normally self-incompatible ;

(e) allowing the recipient plant to mature and assaying the flower parts, especially the pistil and/or the anther, of the mature plant for the expression of the S-locus glycoprotein and optionally the reporter gene; and

(f) selecting those plants which show expression of the S-locus specific glycoprotein and optionally the reporter gene.

Also preferred is a method for obtaining a male and/or female sterile plant which method comprises the following steps :

(a) isolating a tissue specific plant promoter from the reproductive tissue of a donor plant ;

(b) operably linking the tissue specific plant promoter with a DNA sequence that encodes for a toxin protein;

(c) inserting the resulting chimeric gene into the genome of a recipient plant ;

(d) allowing the recipient plant to mature and assaying the flower parts, especially the pistil and/or the anther, of the mature plant for the expression of the toxin gene ; and

(e) selecting those plants which show expression of the toxin gene.

A further embodiment of the present invention is a recombinant DNA molecule that comprises a chimeric genetic construct comprising a structural gene, preferably a structural gene which codes for the production of an S-locus specific glycoprotein from a self-incompatible plant, more preferably a structural gene which is selected from the group consisting of the SLG-13, SLG-14, SLG-22 genes of *Brassica oleracea* and the SLG-8 gene of *Brassica campestris* and most preferably a structural gene which codes for the production of an S-locus specific glycoprotein comprising a DNA sequence as given in sequence listing 1.

Preferred is a recombinant DNA molecule that comprises a chimeric genetic construct wherein the structural gene, preferably a structural gene which codes for the production of an S-locus specific glycoprotein from a self-incompatible plant, more preferably a structural gene which is selected from the group consisting of the SLG-13, SLG-14, SLG-22 genes of *Brassica oleracea* and the SLG-8 gene of *Brassica campestris* and most preferably a structural gene which codes for the production of an S-locus specific glycoprotein comprising a DNA sequence as given in sequence listing 1, is operably linked to a tissue specific plant promoter such that the structural gene is specifically expressed only in the reproductive tissue, preferably in the pistil and/or anther of a recipient plant.

The structural gene can also be a toxin gene. Within the scope of the present invention the term 'toxin gene' is to be understood as comprising such genes that encode for a protein or polypeptide that is capable of conferring a male and/or female sterility to the target plant, selected from the group consisting of nuclease genes such as RNAse or DNAse genes, proteolytic enzyme genes, lectin genes, protein synthesis inhibitor genes, genes that control cell division, lytic peptide genes such as, for example, the thionin gene, the cecropin gene, the maganin gene or the defensin gene, lipase genes, ribozyme genes, antisense genes, phospholipase genes, genes for enzymes in the pathway for systhesis of a phytohormone and genes for an inhibitor of cell wall synthesis.

The present invention further relates to a recombinant DNA molecule that comprises a chimeric genetic construct that besides a structural gene encoding a S-locus glycoprotein further comprises one or more further structural genes, preferably a reporter gene the expression of which in a transgenic cell is indicative of the incorporation of the associated structural gene encoding a S-locus glycoprotein into the transgenic cell's genome and most preferably a reporter gene that is capable of imparting herbicide tolerance. Especially preferred is a gene that is capable of imparting herbicide tolerance to one or more herbicides selected from the group consisting of hygromycin, kanamycin, phosphinotricin, asulam, sulfonylureas, glyphosate, bromoxymil and imidazolinones, wherein the second structural gene is perferably genetically linked with the structural gene coding for the S-locus specific glycoprotein.

A further embodiment of the present invention is a recombinant DNA vector, and especially a recombinant DNA transfer vector comprising :

(a) a structural gene, especially a structural gene which codes for the production of an S-locus specific glycoprotein from a self-incompatible plant, preferably a structural gene which is selected from the group consisting of the SLG-13, SLG-14, SLG-22 genes of *Brassica oleracea* and the SLG-8 gene of *Brassica campestris* and most preferably a structural gene which codes for the production of an S-locus specific glycoprotein comprising a DNA sequence as given in sequence listing 1 ; and

(b) a transformation vector containing DNA sequencesc capable of driving the expression of said structural gene when the construct is incorporated into a plant.

The present invention further relates to a recombinant DNA transfer vector that besides a structural gene encoding a S-locus glycoprotein further comprises one or more further structural genes, preferably a reporter gene the expression of which in a transgenic cell is indicative of the incorporation of the associated structural gene encoding a S-locus glycoprotein into the transgenic cell's genome and most preferably a reporter gene capable of imparting herbicide tolerance, especially herbicide tolerance to one or more herbicides selected from the group consisting of hygromycin, kanamycin, phosphinotricin, asulam, sulfonylureas, glyphosate, bromoxymil and imidazolinones, wherein the gene for herbicide tolerance is genetically linked with the structural gene coding for the S-locus specific glycoprotein.

Also preferred is a recombinant DNA transfer construct comprising the fusion of :

(a) a structural gene which codes for the production of an S-locus specific glycoprotein substantially similar in activity to a S-locus specific glycoprotein gene of *Brassica* sp. ; and

(b) a reporter gene the expression of which in a transgenic cell is indicative of the incorporation of the structural gene into the transgenic cell's genome.

Further preferred within the present invention is a recombinant DNA transfer construct wherein the structural gene is operably linked with a tissue specific plant promoter such that the structural gene is specifically expressed only in the reproductive tissue, preferably in the pistil and/or anther of a recipient plant.

The invention further relates to a self-incompatible plant and the progeny thereof comprising a structural gene which is heterologous with respect to the recipient plant and encodes for the production of an S-locus specific glycoprotein, wherein said plant is not ordinarily self-incompatible.

The present invention further comprises a self-incompatible plant and the progeny thereof comprising a structural gene which encodes for the production of an S-locus specific glycoprotein and said structural gene being genetically linked with one or more further genes, preferably with a reporter gene the expression of which in a transgenic cell is indicative of the incorporation of the associated structural gene encoding a S-locus glycoprotein into the transgenic cell's genome and most preferably a reporter gene capable of imparting herbicide tolerance, especially herbicide tolerance to one or more herbicides selected from the group consisting of hygromycin, kanamycin, phosphinotricin, asulam, sulfonylureas, glyphosate, bromoxymil and imidazolinones, wherein said plant does not ordinarily produce said S-locus specific glycoprotein.

Also comprised are female and/or male sterile plants and the asexually derived progeny thereof that still contains the trait of male and/or female sterility, comprising a structural gene which encodes for the production of a toxin gene, operably linked to a plant specific promoter that is capable of directing the expression of the associated toxin gene in the reproductive tissue, preferably in the anther and/or pistil, of a recipient plant.

A further embodiment of the present invention is a tissue specific plant promoter comprising a DNA sequence comprising a region of an S-locus specific gene that directs the expression of a linked structural gene in the reproductive tissue, preferably in the anther and/or pistil, of a recipient plant.

A more complete understanding of the present invention may be had by reference to the genetic sequence for the SLG gene allele designated as S13 and isolated from *Brassica oleracea*, shown in sequence listing 1. In this sequence, the amino acid translation is also provided for the region of 776 to 2080.

The SLG gene can be differentiated from among other S-related copies by specific hybridization to a probe derived from the 3' end of SLSG-encoding cDNA. Using this 3' gene specific probe (the region 2107 to 2216 in the S13 sequence), it has been possible to isolate SLG genes from a number of different S-allele homozygous genotypes.

Thus, also comprised by the present invention is a DNA probe that specifically hybridizes to the 3' end of an S-locus structural gene, preferably to the 3' end of an S-locus specific structural gene which is SLG-8, SLG-13, SLG-14, SLG-22, and SLG-2 and most perferably to the region 2107 to 2216 in the S13 sequence.

In general, the sequences responsible for the correct tissue and cell type specific and developmental regulation of the SLG genes have been shown to reside in sequences that lie 5' to the transcribed and protein-coding region. Primer-extension experiments have shown that the 5'-end of the mRNA A transcript of the SLG-13 gene lies 15 base pairs 5' to the initiating methionine codon. In addition, transformation experiments have shown that an SLG-13 gene containing only 411 base pairs 5' to the initiating methionine codon is expressed specifically in the pistil of transgenic *Nicotiana* in manner identical to that described for the gene construct containing 4.2 kilobases of 5' sequences. Therefore the promoter sequence is contained in the region extending from position -17 to position -411, more particularly in the region extending from approximately -140 to -411 [position +1 being the A of the initiating ATG].

In addition to the SLG-13 gene sequence given in sequence listing 1, the sequence alignments and differences in the coding region among fow S-locus alleles of *Brassica oleracea* are given in sequence listing 2. The nucleotide sequences were determined from the cDNA inserts of lambdagt10 clones isolated from stigma cDNA libraries. The alignments are further based upon predicted amino acid sequence and nucleotide sequence similarity, attempting to minimize both differences and gaps. Any gaps in the sequence are indicated by dots in the sequence.

To determine if the S-locus glycoprotein gene is expressed in anthers, and to define the site and timing of this expression, the present invention utilizes an E. coli β-glucuronidase (GUS) reporter gene fusion to monitor S-locus glycoprotein gene promoter activity in tissues of developing flowers. It goes without saying that within the present invention any other suitable reporter gene can also be used for that purpose. A chimeric gene is constructed by inserting a 3.65 kb BamHI fragment containing the 5′ upstream region of the S-locus glycoprotein gene, especially a $SLG_{13}$ gene (although other SLG genes described herein may be used as well) derived from Brassica oleracea [see J.B. Nasrallah (1988)] (the 3.65 kb BamHI promoter fragment extends from a BamHI site 3,650 kb upstream of the initiating ATG codon in the S-locus gene isolated from the $S_{13}$ homozygote to a BamHI site introduced 8 bp upstream of the ATG bodon by site-directed mutagenesis) into the GUS expression vector pBI101 [see R.A. Jefferson et al. (1987)]. The resulting vector plasmid, pMKT8 is introduced into Arabidopsis thaliana, a member of the Brassicaceae that has a short generation time, is easily transformed, and in which the self incompatibility trait is not known to occur. However, the rapid flowering of regenerated transgenic plants makes Arabidopsis well suited for the analysis of flower-specific promoters such as those of the S-locus glycoprotein gene. Root segments are inoculated with Agrobacterium cells harboring pMKT8, and transgenic plants are selected based on kanamycin resistance [transgenic plants were produced following infection of Arabidopsis thaliana (L.) Heynh. strain C24 root explants with Agrobacterium tumefaciens as described in PNAS USA 85 :5536 (1988) ; the Agrobacterium strain is pCIB542/A136, a strain derived from helper plasmid pEHA101 containing binary vector pMKT8 ; transformants are selected, and seeds produced by these plants are germinated on medium containing kanamycin in a suitable concentration].

In addition to the reporter gene fusion described above, a second fusion gene construct is made in order to further characterize the sites of S-gene expression as well as to study the function of tissues that express the S-gene. In this construct, the 3.65 kb $S_{13}$ gene promoter fragment is fused to the coding sequence of the Diphtheria toxin A polypeptide in the plant transformation vector pBIN19 [see M Bevan, Nucleic Acids Res. 12 :8711 (1984)] to generate plasmid pMKT17. Diphtheria toxin A polypeptide is known to block protein synthesis through ADP-ribosylation of elongation factor II in many organisms, including plants [see R.J. Collier (1977)], and has also been demonstrated to have nuclease activity [see M.P. Chang et al. (1989)]. These cytotoxic effects allow a single molecule of Diphtheria toxin A polypeptide to cause cell death [see M. Yamizumi et al. (1978)], and so Diphtheria toxin A polypeptide can serve as a very sensitive indicator of gene expression. Gene fusions to Diphtheria toxin A polypeptide have been used to geneticaly direct cell death in a variety of mammalian tissues [see R.R. Behringer et al. (1988)].

The $S_{13}$ :Diphtheria toxin A polypeptide toxic gene fusion can be introduced into tobacco by Agrobacterium-mediated transformation.

In order to ensure the expression of a structural gene in the plant cell, it is advantageous for the coding gene sequence first to be linked in operable manner to expression sequences capable of functioning in plant cells.

The hybrid gene constructions within the scope of the present invention therefore contain in addition to one or more structural gene(s) also expression signals which include both promoter and terminator sequences and other regulatory sequences of the 3′ and 5′ untranslated regions.

Any promoter and any terminator capable of bringing about an induction of the expression of a coding DNA sequence (structural gene) may be used as a constituent of the hybrid gene sequence. Especially suitable are expression signals originating from genes of plants or plant viruses. Examples of suitable promoters and terminators are those of the Cauliflower Mosaic Virus genes (CaMV) such as the 35S and 19S expression signals or homologous DNA sequences that still have the characteristic properties of the mentioned expression signals. Also suitable are bacterial expression signals, especially the expression signals of the nopaline synthase genes (nos) or the octopine synthase genes (ocs) from the Ti-plasmids of Agrobacterium tumefaciens.

In accordance with the invention there may be used as starting material for the 35S transcription control sequences, for example, the Scal fragment of the CaMV strain "S", which includes the nucleotides 6808-7632 of the gene map [Frank G et al (1980)].

The 19S promoter and 5′ untranslated region is located on a genome fragment between the PstI site (position 5386) and the HindIII site (position 5850) of the CaMV gene map [Hohn et al (1982)]. The corresponding terminator and 3′ untranslated region is located on an EcoRV/BglII fragment between positions 7342 and 7643 of the CaMV genome.

Also suitable within the scope of this invention are the expression signals of the CaMV strain CM 1841, the complete nucleotide sequence of which is described in Gardner RC et al (1981).

A further effective representative of a plant promoter that may be used is an over-producing plant promoter. Provided that this type of promoter is operably linked to the gene sequence that codes for a desired gene product, it should be capable of mediating the expression of the said gene sequence.

Over-producing plant promoters that may be used within the scope of the present invention include the promoter of the small subunit (ss) of ribulose-1,5-bisphosphate carboxylase from soybeans and also the promoter of the chlorophyll-a/b-binding protein. These two promoters are known for the fact that they are induced by light in eukaryotic plant cells [see, for example, Genetic Engineering of Plants, An Agricultural Perspective, Cashmore A (1983)].

Preferred within the present invention are tissue specific plant promoters that direct the expression of a linked structural gene in the reproductive tissue, preferably in the anther and/or pistil, of a recipient plant. Expecially preferred is a tissue specific plant promoter comprising a DNA sequence comprising a region of an S-locus specific gene, preferably a region comprising a 3.65 kb BamHI-fragment of the SLG-13 gene, more preferably a region of the SLG-13 gene extending from approximately position -17 to approximately position -411, and most preferably a region of the SLG-13 gene extending from approximately position -150 to approximately position -411 of the SLG-13 gene, which region is capable of directing the expression of an associate d structural gene in the reproductive tissue, preferably in the anther and/or pistil, of a recipient plant.

Besides the promoter region there are further regulatory DNA sequences known that may also be used for the construction of chimeric gene constructs including those sequences, for example, that are capable of regulating the transcription of an associated DNA sequence in plant tissues in the sense of induction or repression.

There are, for example, individual plant genes that are known to be induced by various internal and external factors, such as plant hormones, heat shock, chemicals, pathogens, oxygen deficiency, light, etc..

As an example of gene regulation by a plant hormone, mention should here be made of abscisic acid (ABS), which is known to induce the excess of mRNAs that occurs during the late embryonal phase in cotton. A further example is gibberellic acid (GA3) which induces malate synthase transcripts in castor beans and isoenzymes of α-amylase in the aleurone layers of barley.

The activity of glucanase and chitinase in bean leaves can be markedly increased by treatment with the stress hormone ethylene. In the case of chitinase, this induction effect is controlled *via* the promoter of the chitinase gene, and it was possible to demonstrate this by reporter gene tests using a promoter from the chitinase gene of beans (*Phaseolus vulgaris*).

The regulation of heat-shock-sensitive protein genes of soybeans has been studied in detail. Treating the plants for several hours at a temperature of 40°C results in the *de novo* synthesis of so-called heat-shock proteins. A large number of those genes have since been isolated, and their regulation has been analysed in detail. The expression of those genes is controlled primarily at the transcription level. For example, if the promoter of the hps70 gene is fused with the neomycin phosphotransferase II (NPT II) gene, the chimaeric gene so formed can be induced by a heat shock (Spena *et al*, 1985).

Another class of genes that are inducible in plants comprises the light-regulated genes, especially the nuclear-coded gene of the small subunit of ribulose-1,5-bisphosphate carboxylase (RUBISCO). Morelli *et al* (1985) have shown that the 5'-flanking sequence of a RUBISCO gene from the pea is capable of transferring light-inducibility to a reporter gene, provided the latter is linked in chimaeric form to that sequence. It has also been possible to extend this observation to other light-induced genes, for example the chlorophyll-a/b-binding protein.

The alcohol dehydrogenase genes (adh genes) of maize have been the subject of intensive research. The adh1-s gene from maize was isolated, and it was shown that a part of the 5'-flanking DNA is capable of inducing the expression of a chimaeric reporter gene (e.g. chloramphenicol acetyl transferase ; CAT) when the temporarily transformed tissue was subjected to anaerobic conditions [Howard *et al* (1987)].

A further group of regulatable DNA sequences comprises chemically regulatable sequences that are present, for example, in the PR (pathogenesis-related) protein genes of tobacco and are inducible by means of chemical regulators.

The regulatable DNA sequences mentioned by way of example above may be of both natural and synthetic origin, or they may comprise a mixture of natural and synthetic DNA sequences.

The present invention therefore also includes chimaeric genetic constructions that may contain, in addition to the promoter sequences in operable linkage with a structural gene, further regulatory sections of DNA sequence permitting, for example, specifically controlled induction or repression of gene expression.

The various sections of sequence can be linked to one another by methods known *per se* to form a complete DNA sequence expressible in plant cells. Suitable methods include, for example, the *in vivo* recombination of DNA sequences having homologous sections and the *in vitro* linking of restriction fragments.

As cloning vectors there are generally used plasmid or virus (bacteriophage) vectors having replication and control sequences originating from species that are compatible with the host cell.

The cloning vector generally carries an origin of replication and, in addition, specific genes that lead to phenotypic selection features in the transformed host cell, especially to resistance to antibiotics or to specific herbicides. The transformed vectors can be selected on the basis of those phenotypic markers after transformation in a host cell.

Selectable phenotypic markers that may be used within the scope of this invention include, for example, resistance to ampicillin, tetracycline, hygromycin, kanamycin, methotrexate, G418 and neomycin, but this list, which is given only by way of example, is not intended to limit the subject of the invention.

Suitable host cells within the scope of this invention are prokaryotes, including bacterial hosts, for example A. tumefaciens, E. coli, S. typhimurium and Serratia marcescens, and also cyanobacteria. Eukaryotic hosts, such as yeasts, mycelium-forming fungi and plant cells, may also be used within the scope of this invention.

The splicing of the hybrid gene construction according to the invention into a suitable cloning vector is carried out using standard methods, such as those described in Maniatis et al (1982).

As a rule, the vector and the DNA sequence to be spliced in are first cleaved with suitable restriction enzymes. Suitable restriction enzymes are, for example, those that yield fragments having blunt ends, for example SmaI, HpaI and EcoRV, or enzymes that form cohesive ends, for example EcoRI, SacI and BamHI.

Both fragments having blunt ends and those having cohesive ends that are complementary to one another can be linked again using suitable DNA ligases to form a continuous uniform DNA molecule.

Blunt ends can also be produced by treatment of DNA fragments that have projecting cohesive ends with the Klenow fragment of the E. coli DNA polymerase by filling the gaps with the corresponding complementary nucleotides.

On the other hand, cohesive ends can also be produced by artificial means, for example by the addition of complementary homopolymeric tails to the ends of a desired DNA sequence and of the cleaved vector molecule using a terminal deoxynucleotidyl transferase, or by the addition of synthetic oligonucleotide sequences (linkers) that cry a restriction cleavage site, and subsequent cleavage with the appropriate enzyme.

The cloning vector and the host cell transformed by that vector are generally used to increase the number of copies of the vector. With an increased number of copies it is possible to isolate the vector crying the hybrid gene construction and prepare it, for example, for insertion of the chimaeric gene sequence into the plant cell.

In a further process step, these plasmids or derivatives thereof may be used as transfer vectors to insert the structural genes coding for a desired gene product or non-coding DNA sequences having a regulatory function, for example anti-sense DNA, into the plant cell and, optionally, to integrate them into the plant genome.

Preferred within the scope of the present invention is a recombinant DNA transfer vector comprising :

(a) a structural gene, especially a structural gene which codes for the production of an S-locus specific glycoprotein from a self-incompatible plant, preferably a structural gene which is selected from the group consisting of the SLG- 13,

SLG-14, SLG-22 genes of Brassica oleracea and the SLG-8 gene of Brassica campestris and most preferably a structural gene which codes for the production of an S-locus specific glycoprotein comprising a DNA sequence as given in sequence listing 1 ; and

(b) a transformation vector containing DNA capable of driving the expression of said structural gene when the construct is incorporated into a plant.

Also preferred is a recombinant DNA transfer vector that, besides a structural gene encoding a S-locus glycoprotein, further comprises a gene capable of imparting herbicide tolerance, preferably a gene capable of imparting herbicide tolerance to one or more herbicides selected from the group consisting of hygromycin, kanamycin, phosphinotricin, asulam, sulfonylureas, glyphosate, bromoxymil and imidazolinones, wherein the gene for herbicide tolerance is genetically linked with the structural gene coding for the S-locus specific glycoprotein.

Also preferred is a recombinant DNA transfer construct comprising the fusion of :

(a) a structural gene which codes for the production of an S-locus specific glycoprotein substantially similar in activity to a S-locus specific glycoprotein gene of Brassica sp. ; and

(b) a reporter gene the expression of which in a transgenic cell is indicative of the incorporation of the structural gene into the transgenic cell's genome.

Further preferred within the present invention is a recombinant DNA transfer construct wherein the structural gene is operably linked with a tissue specific plant promoter such that the structural gene is specifically expressed only in the reproductive tissue, preferably in the pistil and/or anther of a recipient plant.

The above described transfer vectors comprising the chimeric genetic constructions may be introduced into the target plant by a variety of processes which have come into existance for introducing DNA into plant cells.

One possible method consists, for example, in bringing plant cells into contact with viruses or with Agrobacterium. This may be achieved by infecting sensitive plant cells or by co-cultivating protoplasts derived from plant cells. Within the scope of this invention, Cauliflower Mosaic Virus (CaMV) may also be used as a vector for the insertion of the chimaeric genetic construction according to the invention into the plant. The total viral DNA genome of CaMV is integrated into a bacterial parental plasmid to form a recombinant DNA molecule that can be propagated in bacteria. After cloning has been carried out, the recombinant plasmid is cleaved, using restriction enzymes, either randomly or at very specific, non-essential sites within the viral part of the recombinant plasmid, e.g. within the gene that codes for the transferability of the virus by aphids, and the hybrid gene construction is cloned into that cleavage site.

A small oligonucleotide, a so-called linker, which has a single, specific restriction recognition site, may also be integrated. The recombinant plasmid so modified is cloned again and further modified by splicing the hybrid gene construction into a restriction site that occurs only once.

The modified viral portion of the recombinant plasmid can then be cut out of the bacterial parental plasmid and used for the inoculation of the plant cells or of the whole plants.

Another method of inserting the chimaeric gene construction into the cell makes use of the infection of the plant cell with *Agrobacterium tumefaciens* and/or *Agrobacterium rhizogenes*, which has been transformed using the said gene construction. The transgenic plant cells are then cultured under suitable culture conditions known to the person skilled in the art, so that they form shoots and roots and whole plants are finally formed.

A further possible method of transforming plant material comprises mixed infection using both *Agrobacterium rhizogenes* and transformed *Agrobacterium tumefaciens*, as described by Petit *et al* (1986) for the transformation of carrots. The mixing ratio must be such that the root colonies formed on the basis of the *A. rhizogenes* transformation also contain a high proportion of *A. tumefaciens* Ti-plasmids. This may be achieved, for example, by applying *A. rhizogenes* and *A. tumefaciens* together to the plant material in known manner in a mixing ratio of from 1 :1 to 1 :100, preferably in a mixing ratio of 1 :10. The transgenic plants are then cultured under suitable culture conditions known to the person skilled in the art, so that they form shoots and roots and whole plants are finally formed. The two *Agrobacterium* species are advantageously not mixed until shortly before the actual inoculation of the plant material to be transformed.

The chimaeric gene construction according to the invention can therefore be transferred into suitable plant cells by means of, for example, the Ti-plasmid of *Agrobacterium tumefaciens* or the Ri-plasmid of *Agrobacterium rhizogenes*. The Ti-plasmid or Ri-plasmid is transferred to the plant in the course of infection by *Agrobacterium* and integrated in stable manner into the plant genome.

Both Ti-plasmids and Ri-plasmids have two regions that are essential for the production of transformed cells. One of those regions, the transfer-DNA (T-DNA) region, is transferred to the plant and leads to the induction of tumours. The other region, the virulence-imparting (vir) region, is essential only for the formation of the tumours, not for their maintenance. The dimensions of the transfer-DNA region can be enlarged by incorporation of the chimaeric gene construction without the transferability being impaired. By removing the tumour-inducing genes and incorporating a selectable marker, the modified Ti- or Ri-plasmid can be used as a vector for the transfer of the gene construction according to the invention into a suitable plant cell.

The vir region causes transfer of the T-DNA region of *Agrobacterium* to the genome of the plant cell irrespective of whether the T-DNA region and the vir region are present on the same vector or on different vectors within the same *Agrobacterium* cell. A vir region on a chromosome also induces the transfer of the T-DNA from a vector into a plant cell.

Within the scope of this invention, therefore, there is preferably used a system for transferring a T-DNA region of *Agrobacterium* into plant cells, in which system the vir region and the T-DNA region are located on different vectors. Such a system is known as a "binary vector system", and the vector containing the T-DNA is accordingly designated a "binary vector".

Any T-DNA-containing vector that can be transferred into plant cells and permits selection of the transformed cells is suitable for use within the scope of this invention.

Especially preferred within the scope of this invention is a shuttle vector that contains the chimaeric genetic construction according to the invention cloned in between the left border sequence (LB) and the right border sequence (RB) and that is capable of stable replication both in *E. coli* and in *A. tumefaciens*.

Using newly developed transformation techniques, it has also become possible to transform *in vitro* plant species that are not natural host plants for *Agrobacterium*. For example, monocotyledonous plants, especially the cereal species and various grasses, are not natural hosts for *Agrobacterium*.

However, it has become increasingly evident that monocotyledons can also be transformed using *Agrobacterium*, so that, using new experimental formulations that are now becoming available, cereals and grass species are also amenable to transformation [Grimsley NH *et al* (1987)].

Preferred within the scope of this invention is so-called leaf disk transformation using *Agrobacterium* [Horsch *et al* (1985)]. Sterile leaf disks from a suitable target plant are incubated with *Agrobacterium* cells containing one of the chimaeric gene constructions according to the invention, and are then transferred into or onto a suitable nutrient medium. Especially suitable, and therefore preferred within the scope of this invention, are LS media that have been solidified by the addition of agar and enriched with one or more of the plant growth regulators customarily used, especially those selected from the group of the auxins consisting of α-naphthylacetic acid, picloram, 2,4,5-trichlorophenoxyacetic acid, 2,4-dichlorophenoxyacetic acid, indole-3-butyric acid, indole-3-lactic acid, indole-3-succinic acid, indole-3-acetic acid and p-chlorophenoxyacetic acid, and from the group of the cytokinins consisting of kinetin, 6-benzyladenine, 2-isopentenyladenine and zeatin. The preferred concentration of auxins and cytokinins is in the range of from 0.1 mg/l to 10 mg/l.

After incubation for several days, but preferably after incubation for 2 to 3 days at a temperature of from 20°C to 40°C, preferably from 23°C to 35°C and more especially at 25°C and in diffuse light, the leaf disks are transferred to a suitable medium for the purpose of shoot induction. Especially preferred within the scope of this invention is an LS medium that does not contain auxin but to which a selective substance has been added for the selection of the transformants. The cultures are kept in the light and are transferred to fresh medium at suitable intervals, but preferably at intervals of one week. Developing green shoots are cut out and cultured further in a medium that induces the shoots to form roots. Especially preferred within the scope of this invention is an LS medium that does not contain auxin or cytokinin but to which a selective substance has been added for the selection of the transformants.

In addition to Agrobacterium-mediated transformation, within the scope of this invention it is possible to use direct transformation methods for the insertion of the gene constructions according to the invention into plant material.

For example, the genetic material contained in a vector can be inserted directly into a plant cell, for example using purely physical procedures, for example by microinjection using finely drawn micropipettes [Neuhaus *et al* (1987)] or by bombarding the cells with microprojectiles that are coated with the transforming DNA ["Microprojectile Bombardment" ; Wang Y-C *et al* (1988)].

Other possible methods for the direct transfer of genetic material into a plant cell comprise treatment of protoplasts using procedures that modify the plasma membrane, for example polyethylene glycol treatment, heat shock treatment or electroporation or a combination of those procedures [Shillito *et al* (1985)].

In the electroporation technique, plant protoplasts together with plasmids that contain the hybrid gene construction are subjected to electrical pulses of high field strength. This results in a reversible increase in the permeability of biomembranes and thus allows the insertion of the plasmids. Electroporated plant protoplasts renew their cell wall, divide and form callus tissue. Selection of the transformed plant cells can take place with the aid of the above-described phenotypic markers.

A further method for the direct introduction of genetic material into plant cells which is based on purely chemical procedures and which enables the transformation to be carried out very efficiently and rapidly is described in Negrutiu I *et al* (1987).

Also suitable for the transformation of plant material is direct gene transfer using co-transformation (Schocher RJ *et al* 1986).

Co-transformation is a method that is based on the simultaneous taking up and integration of various DNA molecules (non-selectable and selectable genes) into the plant genome and that therefore allows the detection of cells that have been transformed with non-selectable genes.

The list of possible transformation methods given above by way of example is not claimed to be complete and is not intended to limit the subject of the invention in any way.

Those cell clones which contain the hybrid gene construction incorporated in their genome are selected using customary selection, screening and detection methods and are used for the regeneration of transgenic plants.

The regeneration of protoplasts kept in culture to form whole plants is described, for example, in Potrykus I and Shillito RD (1986).

The regeneration processes differ from one plant species to another. In general, however, protoplasts in one of the known culture media are stimulated to divide and form cell walls. There are finally formed callus cultures which can be induced to form roots or shoots by treatment with specific active agents, for example auxins and cytokinins.

The plantlets so obtained can then be transferred to soil and cultivated further in the same manner as normal seedlings.

Efficient regeneration depends especially upon the medium, the genotype and the previous history of the culture. If these three variables are adequately controlled, the regeneration is completely reproducible and repeatable.

The regenerated transgenic plants, which contain a structural gene, expressible in the plant cell, of the above-described hybrid gene construction as an integral component of the plant genome, can be propagated vegetatively, preferably in the form of sterile shoot cultures.

The stable integration of an operative expressible gene into the plant genome of the regenerated transgenic plants is verified by reference to the mitotic stability of the integrated gene and on the basis of its behaviour as Mendelian characteristic during meiosis and using Southern blot analysis (Southern EM, 1975).

The broad concept of this invention therefore also includes transgenic plant material, selected from the group consisting of protoplasts, cells, callus, tissues, organs, seeds, embryos, pollen, ovules, zygotes etc., including whole plants, but especially whole, fertile plants, that has been transformed by means of the processes described above and contains the recombinant DNA according to the invention in expressible form, and processes for the preparation of said transgenic plant material.

The present invention therefore relates also to the production of recipient plants that at least contain the said structural gene incorporated in their genome.

Preferred within the scope of the present invention are self-incompatible plants comprising a structural gene which is heterologous with respect to the recipient plant and encodes for the production of an S-locus specific glycoprotein, wherein said plant is not ordinarily self-incompatible.

Especially preferred is a self-incompatible plant comprising a structural gene which encodes for the production of a
n S-locus specific glycoprotein and said structural gene is genetically linked with a gene capable of imparting herbicide tolerance, perferably with a gene capable of imparting herbicide tolerance to one or more herbicides selected from the group consisting of hygromycin, kanamycin, phosphinotricin, asulam, sulfonylureas, glyphosate, bromoxymil and imidazolinones, wherein said plant does not ordinarily produce said S-locus specific glycoprotein.

Also preferred are female and/or male sterile plants comprising a structural gene which encodes for the production of a toxin gene, operably linked to a plant specific promoter that is capable of directing the expression of the associated toxin gene in the reproductive tissue, preferably in the anther and/or pistil, of a recipient plant.

A more complete understanding of the present invention may be had by reference to the following figures and examples which are presented to exemplify, not limit, the present invention. In the figures ;

Figure 1 is a restriction map of the EcoR1 fragment containing the S13 SLG gene and showing the derivation of the 3' probe specific to the SLG gene. The stippled box represents the SLSG protein-coding region. The black bar indicated by the arrowhead corresponds to the 3' untranslated region from which the gene-specific probe is derived. B = BamH1 ; E = EcoR1 ; H = HindIII ; S = Sal1 ; X = XhoI.

Figure 2 is a restriction map of genes SLG-8, SLG-14, SLG-22 and SLG-2. B = BamH1 ; E = EcoR1 ; H = HindIII ; Sp = Sph1 ; St = Sst1 ; X = Xbal ; K = Kpn1, and the orientation is in the 5'----- 3'direction.

Figure 3 is a map of SLR2-cDNA isolated from 2 and S15 genotypes. B = BamH1 ; Bg = Bg12 ; E = EcoR1; and St = Sst1.

Figure 4 is a summary of the histochemical detection of GUS activity in the anther and stigma of trangenic *Arabidopsis*.

NON-LIMITING EXAMPLES :

General recombinant DNA techniques :

Since many of the recombinant DNA techniques employed in this invention are a matter of routine for the person skilled in the art, it is better to give a short description of these generally used techniques here rather than to describe them every time they occur. Except where there is a specific indication to the contrary, all these procedures are described in the Maniatis *et al* (1982) reference.

A. Cleaving with restriction endonucleases

A reaction batch typically contains about 50 to 500 μg/ml of DNA in the buffer solution recommended by the manufacturer, New England Biolabs, Beverly, MA.. 2 to 5 units of restriction endonucleases are added for each μg of DNA and the reaction batch is incubated for from one to three hours at the temperature recommended by the manufacturer. The reaction is terminated by heating at 65°C for 10 minutes or by extraction with phenol, followed by precipitation of the DNA with ethanol. This technique is also described on pages 104 to 106 of the Maniatis *et al* (1982) reference.

B. Treatment of the DNA with polymerase in order to produce blunt ends

50 to 500 μg/ml of DNA fragments are added to a reaction batch in the buffer recommended by the manufacturer, New England Biolabs. The reaction batch contains all four deoxynucleotide triphosphates in concentrations of 0.2 mM. The reaction takes place over a period of 30 minutes at 15°C and is then terminated by heating at 65°C for 10 minutes. For fragments obtained by cleaving with restriction endonucleases that produce 5'-projecting ends, such as EcoRI and BamHI, the large fragment, or Klenow fragment, of DNA polymerase is used. For fragments obtained by means of endonucleases that produce 3'-projecting ends, such as PstI and

13

Sacl, the T4-DNA polymerase is used. The use of these two enzymes is described on pages 113 to 121 of the Maniatis *et al* (1982) reference.

## C. Agarose gel electrophoresis and purification of DNA fragments from gels

Agarose gel electrophoresis is carried out in a horizontal apparatus, as described on pages 150 to 163 of the Maniatis *et al.* reference. The buffer used is the tris-borate buffer described therein. The DNA fragments are stained using 0.5 mg/ml of ethidium bromide which is either present in the gel or tank buffer during electrophoresis or is added after electrophoresis. The DNA is made visible by illumination with long-wave ultraviolet light. If the fragments are to be separated from the gel, an agarose is used that gels at low temperature and is obtainable from Sigma Chemical, St. Louis, Missouri. After the electrophoresis the desired fragment is cut out, placed in a plastics test tube, heated at 65'C for about 15 minutes, extracted three times with phenol and precipitated twice with ethanol. This procedure is slightly different from that described by Maniatis *et al* (1982) on page 170.

As an alternative, the DNA can be isolated from the agarose with the aid of the Geneclean kit (Bio 101 Inc., La Jolla, CA, USA).

## D. Addition of synthetic linker fragments to DNA ends

If it is desired to add a new endonuclease cleavage site to the end of a DNA molecule, the molecule is optionally first treated with DNA-polymerase in order to produce blunt ends, as described in the above section. About 0.1 to 1.0 µg of this fragment is added to about 10 µg of phosphorylated linker DNA, obtained from New England Biolabs, in a volume of 20 to 30 µl with 2 µl of T4 DNA ligase from New England Biolabs, and 1 mM ATP in the buffer recommended by the manufacturer. After incubation overnight at 15°C, the reaction is terminated by heating at 65°C for 10 minutes. The reaction batch is diluted to about 100 µl in a buffer appropriate for the restriction endonuclease that cleaves the synthetic linker sequence. About 50 to 200 units of this endonuclease are added. The mixture is incubated for 2 to 6 hours at the appropriate temperature, then the fragment is subjected to agarose gel electrophoresis and purified as described above. The resulting fragment will then have ends with endings that were produced by cleaving with the restriction endonuclease. These ends are usually cohesive, so that the resulting fragment can then readily be linked to other fragments having the same cohesive ends.

## E. Removal of 5'-terminal phosphates from DNA fragments

During the plasmid cloning steps, the treatment of the vector plasmid with phosphatase reduces the recircularisation of the vector (discussed on page 13 of the Maniatis *et al* reference). After cleavage of the DNA with the correct restriction endonuclease, one unit of calf intestinal alkaline phosphatase obtained from Boehringer-Mannheim, Mannheim, is added. The DNA is incubated at 37°C for one hour and then extracted twice with phenol and precipitated with ethanol.

## F. Linking of the DNA fragments

If fragments having complementary cohesive ends are to be linked to one another, about 100 ng of each fragment are incubated in a reaction mixture of 20 to 40 µl containing about 0.2 unit of T4 DNA ligase from New England Biolabs in the buffer recommended by the manufacturer.

Incubation is carried out for 1 to 20 hours at 15°C. If DNA fragments having blunt ends are to be linked, they are incubated as above except that the amount of T4 DNA ligase is increased to 2 to 4 units.

## G. Transformation of DNA into *E. coli*

*E. coli* strain HB101 is used for most of the experiments. DNA is introduced into *E. coli* using the calcium chloride process, as described by Maniatis *et al* (1982), pages 250 and 251.

## H. Screening of *E. coli* for plasmids

After transformation, the resulting colonies of *E. coli* are tested for the presence of the desired plasmid by means of a rapid plasmid isolation process. Two customary processes are described on pages 366 to 369 of the Maniatis *et al* (1982) reference.

## I. Large-scale isolation of plasmid DNA

Processes for the isolation of plasmids from *E. coli* on a large scale are described on pages 88 to 94 of the Maniatis *et al* (1982) reference.

## J. Cloning in M13 phage vectors

In the following description it is to be understood that the double-strand replicative form of the phage M13 derivatives is used for routine processes, such as cleaving with restriction endonuclease, linking etc..

Unless there is a specific indication to the contrary, enzymes can be obtained from Boehringer, Biolabs (BRL). They are used in accordance with the manufacturer's instructions unless otherwise indicated.

## K. Southern blot analysis

The extracted DNA is first treated with restriction enzymes, then subjected to electrophoresis in a 0.8 % to 1 % agarose gel, transferred to a nitrocellulose membrane [Southern EM (1975)] and hybridised with the DNA to be detected which has previously been subjected to nick-translation (DNA-specific activities of $5 \times 10^8$ to $10 \times 10^8$ c.p.m./$\mu$g). The filters are washed three times for 1 how each time with an aqueous solution of 0.03M sodium citrate and 0.3M sodium chloride at 65°C. The hybridised DNA is made visible by blackening an X-ray film over a period of 24 to 48 hours.

## L. Western blot analysis

After SDS-polyacrylamide gel electrophoresis, the proteins are transferred electrophoretically to a nitrocellulose or nylon filter. This filter is then first pre-treated with a blocking agent (for example 5 % skim milk powder in PBS : in the following referred to as milk/PBS). The filter is then incubated for several hours with an antiserum that reacts with the compound to be detected. The filter pre-treated in this manner is washed several times with milk/PBS and then incubated with a commercially available secondary antibody that is coupled to an enzyme [for example peroxidase-coupled goat anti-rabbit antibody (BIORAD), 1 :2000 diluted in milk/PBS]. The filter is again washed in PBS and then stained with chloronaphthol and hydrogen peroxide in accordance with the manufacturer's [BIORAD] instructions. Further details are given in Sambrook *et al* (1989).

## M. Northern blot analysis

Total RNA can be prepared in accordance with the method described in Logemann *et al* (1987), but it is advantageous to introduce an additional precipitation step with 2M LiCl in order to eliminate any residues of contaminant DNA. The RNA (0.4 mg or 4 mg) is then fractionated by electrophoresis in a 1 % agarose gel containing 6.7 % formaldehyde [Maniatis *et al* (1982)]. The individual fractions are transferred to 'Zeta-Probe' membranes [BIORAD] and hybridised with a $^{32}$P-labelled probe. The size of the hybridising DNA can be estimated by reference to the standard run at the same time.

## Example I : Vector construction for transformation of self- Incompatibility into plants

### (a) Construction of pCIB 542, an agropine VIR helper plasmid bearing a spectinomycin drug resistance gene in the plane of the T-DNA

The Ti plasmid, pTi Bo 542 [Sciaky, D., Montoya, A.L. & Chilton, M.-D. (1978)], is of interest because *Agrobacteria* bearing this Ti plasmid are able to infect the agronomically important legumes alfalfa and soybean [Hood, E.E., *et al.* (1984)]. The construction of a pTi Bo 542 derivative deleted of the T-DNA has been described [Hood, E.E., *et al.* (1986)]. In this construction, named EHA 101, the T-DNA is replaced by the a kanamycin drug resistance gene.

A derivative of EHA 101 having the kanamycin drug resistance gene replaced by a spectinomycin drug resistance gene is constructed as follows : The plasmid p pi delta 307 [Hood, E.E., *et al.* (1986)] has a 1.7 kb region of homology to the left side of Bam 5 of pTi Bo 542 [Hood, E.E., *et al.* (1984)] and an 8 kb region of homology to the right side of Bam2a of pTi Bo 542, separated by a unique EcoR1 site. The plasmid pMON 30/SR20, which has been deposited under the Budapest Treaty with ATCC in Rockville, Maryland and is designated with accession number 67113, bears the spectinomycin/streptomycin [spc/str] drug resistance gene from Tn7 [Hollingshead, S. and Vapnek, D. (1985)] pMon 30 is digested with EcoR1, the 5.5 kb fragment containing the spc/str gene isolated from an agarose gel, and ligated into EcoR1-restricted p pi delta 307. The desired recombinant is selected as a spectinomycin resistant (50 ug/ml) tetracyline resistant (10 ug/ml) transformant of *E. coli* HB 101.

Plasmid DNA is introduced into agrobacteria A136/EHA101 by transformation. The desired transformant is selected by its streptomycin-resistant tetracycline-resistant kanamycin-resistant phenotype. Homogenotes [Matzke, A.J.M. & Chilton, M.-D. (1981)] of EHA101 and the spectinomycin plasmid are selected after conjugal introduction of the eviction plasmid R751-pMG2 [Jacoby, G. *et al.* (1976)] and selection on gentamycin (50 µg/ml) and spectinomycin. The desired homogenote has a gentamycin-resistant spectinomycin-resistant tetracycline-sensitive kanamycin-sensitive phenotype. The structure of the resulting plasmid is confirmed by Southern analysis.

A derivative cured of the eviction plasmid is then selected by its drug resistance phenotype of gentamycin sensitivity. This selection is confirmed by Southern analysis.

### (b) Construction of transformation vectors

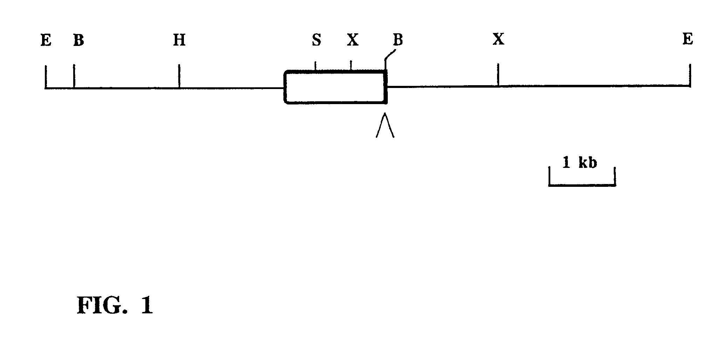

The *Brassica* gene used in the transformation according to one embodiment of the present invention is designated SLG-13. It encodes the S-locus specific glycoprotein (SLSG) and is isolated from *Brassica oleracea* $S_{13}$ homozygote. The method for the isolation of this gene is described in Nasrallah *et al.* (1988). SLG-13 is contained on an 11 kilobase (kb) EcoR1 restriction fragment which contains the SLSG-coding region and approximately 4.2 kb of 5' and 5.0 kb of 3' flanking sequence. (See Figure 1).

This EcoR1 fragment is inserted into the multiple cloning site of the binary vector pC1B10 [Rothstein *et al* (1987)] which carries a chimeric kanamycin resistance gene for selection of transformed plants. The recombinant vector pH1 [Moore HM and Nasrallah JB (1990)] or SLG-13/pCIB10 carrying the $S_{13}$ gene is mobilized into *Agrobacterium tumefaciens* strain pC1B542/A136 [a derivative of strain EHA101 described in Hood *et al* (1986) ; see example I(b)] by triparental mating according to standard published procedures [Rogers SG *et al*, (1988)].

Transformation vectors carrying SLG genes are also constructed in the Ti plasmids pBI 121 [which is commercially available from Clonetech Laboratories] and pBIN 19 [Jefferson RA *et al* (1987) ; Bevan M (1984)]. Plant transformation with these vector has also been successful.

Transformation of *Brassica* is performed according to a method used in the related crucifer *Arabidopsis* with a mixture of *A. tumefaciens* cells harboring the SLG13/pCIB10 construct and of root-inducing plasmid from the wild type *A. rhizogenes* strain A4 [Van Sluys *et al* (1987)]. Tissue explants of *B. napus* var. "Westar" are obtained and treated essentially as described by Fry *et al* (1987)]. In our experience, *Brassica* transformation is inefficient. Following selection on kanamycin, one kanamycin-resistant primary transformant, plant 17B-1, is obtained and analyzed for the integration and expression of the S13 transgene.

More specifically, this transformation is performed according to the procedure in Example II.

### Example II : Transformation of Brassica napus

Terminal internodes from a flowering stem of *Brassica napus* var/ "Westar" are removed and surface sterilized in 70 % (v/v) ethanol for 1 minute, 2 % (v/v) sodium hypochlorite for 20 minutes, and followed by a rinse, three times, in sterile distilled water. The sterilized stems are then cut into 5 mm discs and dipped into an *Agrobacterium* cell mixture for 1 minute. The discs are then removed, blotted dry on sterilized filter paper, and placed over a feeder layer of tobacco cells plated onto a co-cultivation medium consisting of MS salts and vitamins, 3 % sucrose, 1 mg/1 NAA, 0.5 mg/1 BAP, and 0.8 % Agar. Following co-cultivation for two days in the dark at 28°C, the discs are transferred to culture plates containing the same medium supplemented with 500 mg/l carbencillin to inhibit bacterial growth and 50 mg/l kanamycin to select for transformation with the SLG-13/pC1B10 construct. The plates are cultured at 25°C under cool white light, and the stem discs are transferred to fresh selective media every two weeks. Kanamycin-resistant plantlets are selected from the culture plates and transferred to rooting medium. After roots developed, the plantlets are potted in soil, grown to maturity, and analyzed for successful transformation.

As part of the analysis of transformed plants, the stable integration of the SLG gene in transformed *Brassica napus* is examined. In view of the presence in the *B. napus* genome of several copies related to the SLG gene, the S13 transgene is most easily visualized by hybridization to the gene-specific probe derived from the 3' untranslated region of SLSG-cDNA (Figwe 1). DNA blot analysis (according to Example III) with this probe revealed the presence of the 11 kb EcoR1 fragment in DNA from 17B-1 but not in DNA from the untransformed "Westar" recipient. Thus no gross rearrangements of the introduced fragment had occurred during transformation and regeneration.

16

## Example III : DNA blot analysis

Nuclear DNA is prepared from leaf tissue according to published techniques [see, for example, Bingham PM *et al* (1981)]. Restriction fragments are fractionated on 0.9 % agarose and transferred to Gene Screen Plus membrane [Gene Screen Plus is sold by Dupond/NEN, Dupont Company, Biotechnology Systems, Wilmington, Deleware, USA]. The blot is prehybridized and hybridized at 65°C in 10 % (w/v) dextran sulfate, 330 mM sodium phosphate pH 7.0, 10 mM EDTA, 5 % (w/v) sodium dodecyl sulfate and salmon testes DNA [Sigma Chemical Company, St. Louis, Missouri, USA] at 100 µg/ml. Hybridization is with the 3'probe labeled with $^{32}$P by random priming. The blot is washed in 0.3 M NaC1, 40 mM Tris-HC1 pH 7.8, 2 mM EDTA and 0.5 % (w/v) sodium dodecyl sulfate at 65°C, and exposed to Kodak XAR-5 film at -70°C with an intensifying screen.

The stable integration of the S13 gene into the genome of the primary transformant is demonstrated by the analysis of progeny plants obtained by selfing and by backcrossing to the "Westar" line. DNA is isolated at the seedling stage from twenty-one selfed progeny plants and twenty backcross progeny plants. All twenty-one selfed plants are shown by DNA blot analysis to carry the S13 gene, indicating integration at more than one site in 17B-1. This conclusion is supported by the segregation of kanamycin-resistance, a trait for which a larger population is examined. Out of 72 selfed progeny plants assayed, 4 plants are kanamycin-sensitive, giving a ratio with close fit into the 1 :15 (sensitive :resistant) ratio expected for two integration events. Furthermore, among 20 progeny plants derived from backcrossing 17B-1 to "Westar", four plants are shown to lack the S13 restriction fragment on DNA blots and to have no detectable SLSG in the stigma. Here again, these numbers approximate the 3 :1 backcross ratio expected for two integration events for transforming DNA. The Medelian ratios obtained in the backcross of 17B-1 to "Westar" also indicate that the ploidy level is correctly maintained dig tissue culture.

Consistent with the interpretation of two integration sites are the results of the analysis of restriction digests of genomic DNA isolated from the recipient "Westar" line, the transgenic plant and four of its selfed progeny. Following digestion with EcoR1, the DNA from all four progeny plants is shown to contain the 11 kb fragment carrying the S13 gene. After digestion with HindIII, a restriction enzyme which cuts once within the 11 kb EcoR1 fragment (see Figure 1), two restriction fragments are detected in the transgenic plant and its progeny, and are missing in "Westar" DNA, again consistent with two sites of integration of the S13-containing restriction fragment.

Plant 17B-1 exhibited phenotypic features characteristic of Ri-transformants namely wrinkled leaves, extruded stigmas and loss of apical dominance. All twenty-one progeny plants derived from 17B-1 exhibited the wrinkled-leaf phenotype at the seedling stage. Thus Ri DNA had stably integrated into the 17B-1 genome. In the population of 20 plants obtained by backcrossing 17B-1 to "Westar", two plants are recovered which had normal leaves and are thus apparently free of Ri plasmid. Based on DNA blot hybridization, these two plants are shown to carry the S13 gene, indicating that the Ri and Ti plasmids had inserted on different chromosomes, and that in future generations transgenic plants cured of Ri plasmid can be recovered.

The expression of the S13 gene in the transgenic *Brassica napus* plants is also studied to confirm that authentic SLSG is being synthesized by the plant. This is possible because the SLSG encoded by the SLG gene in the *B. oleracea* S13 homozygote (the donor plant) has recently been shown to react with MAb/H8, a monoclonal antibody raised against purified SLSG and specific for a protein epitope of SLG6 [Kandasamy MK *et al* (1989)]. Stigmas of the "Westar" recipient plant on the other hand are not known to synthesize SLSG, and in any case, they lack a protein with the electrophoretic properties of S13-SLSG, and show no crossreactivity with anti-SLSG antibody. Electrophoretic (according to Example IV) and immunoblotting (according to Example V) analysis of tissue extracts from plant 17B-1, showed the presence of S13-SLSG in stigmas. No antigenic proteins are detected in the style, ovary or leaf. Furthermore, in 17B-1 stigmas, a protein band with the same mobility as S13-SLSG is evident following sodium dodecyl sulfate-polyacrylamide gel electrophoresis (SDS-PAGE), immunoblotting of 17B-1 stigma extracts revealed a pattern characteristic of SLSG and consisting of a cluster of three species with a molecular weight identical to that of S13-SLSG. Stigmas from twenty different inflorescences of 17B-1 are tested by immunoblotting following SDS-PAGE and found to produce SLSG, indicating that the plant is not chimeric.

## Example IV : Protein gel electrophoresis

Stigmas are excised into liquid nitrogen, pulverized in microcentrifuge tubes with an adapted Teflon tissue homogenizer and resuspended in glass-distilled water for isoelectric focusing (IEF) and in 10 mM Tris-HC1, pH 7.2 for SDS-PAGE. The extracts are clarified by centrifugation and total protein content is determined using the Coomassie protein assay (Pierce). Samples containing 50 µg of total protein are subjected to IEF for 2 hours on pH 3.5-9.5 polyacrylamide gel slabs. For SDS-PAGE, samples are mixed with an equal volume of a solution containing 160 mM Tris-HC1 pH 6.8, 2 % (w/v) SDS, 3 % (w/v) dithiothreitol, 20 % (v/v) glycerol and separated on 10 % (w/v) polyacrylamide gels according to Dreyfuss *et al* (1984). Molecular weight markers are a mixture of six reference proteins.

## Example V : Immunoblotting

Following electrophoresis, transfer to nitrocellulose is by capillary action for IEF gels and by electroblotting according to published techniques for SDS gels [Towbin H *et al* (1975)]. For immunostaining, the nitrocellulose membranes with bound protein are blocked with 1 mg/ml BSA in 10 mM Tris-HC1 pH 7.2, 0.9 M NaC1 and 0.05 % (v/v) Tween-20. The membranes are then treated with MAb/H8, a monoclonal antibody produced against purified SLSG and directed against a protein epitope of SLSG, followed by a treatment with alkaline phosphatase-conjugated anti-mouse IgG (Promega). Immunoreactive components are visualized using BCIP/NBT as substrate.

The level of SLSG produced by 17B-1 is determined as a function of stigma development and relative to that produced in stigmas of the *B. oleracea* S13 homozygote. For developmental analysis, aliquots containing equivalent amounts of total stigma protein extracted from developing flower buds and flowers are spotted onto nitrocellulose, the blots are stained with MAb/H8 and quantitated by densitometry. Data collected indicates that the transgene-encoded SLSG is detected at three days prior to anthesis, its level increased approximately 7-fold as the stigma matured, and reached a maximum at one day prior to anthesis.

For the relative quantitation of SLSG produced by 17B- 1 and by the *B. oleracea* S13 homozygote, aliquots of a dilution series of total stigma protein extracted from open flowers are used as described in Example VI. The data compiled from different extractions and dilution series indicated that 17B-1 stigmas contained only 60 + 11 % of the level of SLSG found in open flower stigmas of the *B. oleracea* S13 homozygote.

## Example VI : Quantitative analysis of SLSG

For quantitative analysis of SLSG antigen, the total soluble protein content of stigma extracts is measured with the Coomassie reagent (Pierce). For developmental analysis, equivalent amounts of total protein isolated from stigmas at different stages of development are used. In order to determine the relative amounts of SLG in 17B-1 and the *B. oleracea* S13 homozygote, a dilution series of total stigma proteins is prepared, and aliquots containing amounts of total protein ranging from 0.001 µg to 1 µg are spotted onto a nitrocellulose membrane using the Hybri-slot apparatus. Following immunostaining, densitometric scanning is performed with a spectrophotometer equipped with gel transport and film holder accessories.

The papillar-cell specific expression of the SLSG gene is transgenic *Brassica napus* is also studied. The spatial distribution of SLSG in transgenic stigmas is analyzed by indirect immunogold labeling of fixed sections using light and electron microscopy. Of the various cells of the stigma only the papillar cells of 17B-1 stigmas stained positively for SLSG following silver enhanced immunogold staining with MAb/H8. The underlying cells of the stigma and cells of the style failed to stain. Comparable sections from "Westar" stigmas which are subjected to the same staining and development procedures failed to stain with the antibody, and are used as controls for both light and electron microscopic immunolocalization. At the ultrastructural level, indirect immunogold labeling resulted in the absence of gold particles over sections prepared from an untransformed "Westar" plant. In plant 17B-1, only the papillar cells of the stigma showed labeling ; the highest concentration of gold particles is ovserved over the wall of these cells. A few gold particles are also detected over the cytoplasmic membranes. Immunolabeling of 17B-1 stigma sections consistently resulted in a lower density of gold particles as compared to the *B. oleracea* S13 homozygote.

The fertility of 17B-1 gametes is assayed in reciprocal crosses to "Westar" and other lines by removing pollen from newly dehisced anthers and applying it to the stigmas of the female plant. The pollinated stigmas are stained with decolorized aniline blue and pollen development is monitored by fluorescent microscopy. It is found that, when used as the male parent, 17B-1 had fully functional pollen which produced normal seed set of 20-30 seed per silique. However, the reciprocal crosses revealed some ovule sterility and resulted in only 3-4 seeds per silique on average.

The compatibility/incompatibility phenotype of the transgenic plant is analyzed by microscopic monitoring of pollen tube behavior following pollination. In this assay, the germination and growth of a large number of pollen tubes into the stigma is indicative of a compatible interaction, while the absence of developed pollen tubes is indicative of an incompatible interaction. Self-pollinations and reciprocal cross-pollinations to the "Westar" cultivar are performed, and pollen tube development at the stigma surface is monitored by fluorescence microscopy at different times ranging from 1 to 12 hours after pollination. The determination of compatibility relationships with the *B. oleracea* S13 homozygote is complicated by the factor of inter-specific incompatibility.

Table 1 shows that plant 17B-1 is reciprocally cross-compatible with the *B. napus* "Westar" cultivar based on the dense pollen tube growth into the stigma at delayed invasion of the stigma by pollen tubes. Initially and up to three hours following pollination, only few pollen tubes invaded the stigma. In 10 different pollinations, between 4 and 57 invading pollen tubes are observed, giving an average of 25 + 19 pollen tubes per stigma. At six hours following self-pollination however, confluent pollen-tube growth is observed.

Table 1. Pollination response of transgenic plant 17B-1 upon self-pollination and in crosses to ""Westar"".

| pollen | 17B-1 | | "Westar" | |
|--------|-------|------|----------|------|
| stigma | 3hr | 6hr | 3hr | 6hr |
| 17B-1 | 25+19* | + | + | + |
| "Westar" | + | + | + | + |

\* : average number of pollen tubes per stigma.

+ : indicates that innmuerable tubes are observed.

As with the sporophytic *Brassica*, self-incompatibility in the gametophytic species, *Nicotiana alta* is also controlled by a single, multiallelic locus, although the gene sequences of between the two are not similar. Whereas the SLG genes in *Brassica* are expressed specifically in the papillar cell of the stigma, the site of inhibition of pollen germination and pollen tube growth in incompatible pollinations, the S-associated genes in *Nicotiana* are expressed throughout the pathway of pollen tube growth, in the secretory cells of the stigma, the transmitting tissue of the style and the placental epidermis of the ovary.

When cloned *B. oleracea* SLG gene is introduced into the readily transformable and self-compatible species of *N. tabacum*, the gene expressed itself at the RNA and protein levels. Furthermore, this expression is seen to be developmentally regulated.

## Example VII : Transfer of SLG gene into Nicotiana

### (a) Transgenic plants

Triparental mating is performed using the pH1 binary vector plus pRK2013 [Ditta G *et al* (1980)] and pCIB542/A136. Leaf strips of axenic plantlets of *N. tabacum* are transformed using standard methods. Kanamycin resistant shoots are rooted, transplanted to soil and grown to maturity in Environmental Growth System chambers at 70°F with a 16h/8h light/dark cycle. Non-transformed plants regenerated from tissue culture of untreated leaf strips are also grown in these growth chambers.

Tissues for DNA, protein or RNA preparation are dissected from individual transgenic plants, frozen in liquid nitrogen and stored at -70°C until use.

### (b) Stages of floral development

Stages of floral development in *N. tabacum* are established by collecting bud length data from 10 mm buds to flowers several days after anthesis. On average, buds 5 days (d) before flowering are 13 mm long ; buds 3 d before flowering are 25 mm long ; buds 2 d before flowering are 40 mm ; bud 1 d before flowering are 48 mm. Flowers are 45 mm on the first day they opened and are not collected for study after this point.

### (c) DNA and RNA analysis

DNA is prepared from leaves of transgenic plants by a miniprep procedure or by nuclear DNA extraction and CsCl purifiction. DNA is cut to completion with restriction enzymes, fractionated on 0.6 and 0.7 % (w/v) agarose gels and transferred to Gene-Screen Plus membrane after denaturation. RNA is prepared from leaf, stigma, style and anther tissue by SDS-proteinase K extraction. Total RNA is denatured with glyoxal, electrophoresed on 1 % (w/v) agarose gels and transferred to Gene-Screen membrane.

Concentration of DNA and RNA solutions is determined by spectrophotometry. Hybridization for both DNA and RNA blots is performed at 67°C using a solution containing 10 % (w/v) dextran sulfate, 0.3 M Sodium Phosphate (pH 7.0), 5 % (w/v) SDS, 10 mM EDTA, and 0.14 mg/ml denatured salmon sperm DNA. The hybridization probe is a cDNA insert from pBOS13 [Nasrallah JB et al(1988)] labeled with $^{32}$P-dATP using a random-primer kit. Levels of hybridization signals in different samples are quantitated using a Quick Scan scanning densitometer.

(d) Protein gel blot analysis

Soluble proteins are extracted from leaf, stigma, style and anther tissue in 100 mM Tris-HC1 buffer pH 7.6. Protein concentrations are determined using a bicinchoninic acid/copper sulfate assay system [available from Pierce, P.O. Box 117, Rockford, Illinois, USA]. Prior to fractionation on SDS-PAGE, protein is denatured at 100°C for five minutes in a solution containing 100 mM dithiothreitol, 2 % (w/v) SDS, 80 mM Tris HC1 pH 6.8, 10 % (v/v) glycerol and bromphenol blue. Electrophoresis is performed at 20 milliamps and 2°C, using gels of 10 % polyacrylamide and 0.1 % SDS, and electroblotted to nitrocellulose. Protein gel blots are stained with the anti-SLSG monoclonal antibody using the Protoblot Western Blot Alkaline Phosphatase system [Promega, 2800 Woods Hollow Road, Madison, Wisconsin, USA].

(e) Immunolocalization

S-protein in flowers of transgenic plants is visualized in situ at the light microscope level using silver-enhanced immunogold labeling. Whole immature buds or dissected stigma/style and anther tissue of transgenic plants are fixed for 2 h in 4 % paraformaldehyde in 0.1 M phosphate buffer pH 7.0, dehydrated and embedded in Paraplast Plus media [Monject Scientific, a Division of Sherwood Medical, St. Louis, Missouri, USA]. Ten micron longitudinal sections are cut and mounted using an egg albumin adhesive. Sections are treated sequentially in phosphate buffered saline (10 mM sodium phosphate pH 7.2, 150 mM sodium chloride) containing one of the following : 1) 5 % (v/v) normal goat serum (Sigma) and 5 % (w/v) Bovine Serum Albumin ; 2) anti-SLSG antibody, 1 :100 in 0.1 % (w/v) BSA ; 3) gold-conjugated goat anti-mouse antisera in 0.1 % (w/v) BSA and 5 % (v/v) gelatin. Sections are incubated in each solution for one hour at room temperature ; rinses in phosphate buffered saline followed the antibody incubations. Two cycles of silver enhancement are performed using InstenSe II reagents [Janssen Life Science Products, a division of Amersham, 2636 South Clearbrook, Arlington Heights, Illinois, USA].

Twenty-three kanamycin-resistant plants from Example VI are grown to matrity along with nineteen non-transformed plants obtained by leaf disk culture. No morphological differences are observed between the transformed and non-transformed plants. All plants set seed when flowers are selfed by hand pollination and are judged self-compatible on this basis. Twelve kanamycin-resistant plants are selected for furhter study of the integration and expression of the SLG gene.

DNA purified from leaves of kanamycin-resistant plants is digested with restriction endonucleases, separated by agarose gel electrophoresis, and blotted to nylon membrane. Hybridization analysis is performed using a labeled cDNA probe, pBOS13, which encodes the B. oleracea S13 SLSG. No Hybridization to the pBOS13 probe is detected in DNA from non-transformed N. tabacum under the hybridization conditions used. DNA from two kanamycin-resistant plants did not hybridize to the pBOS13 probe and did not express the SLG gene. Ten kanamycin-resistant plants had DNA fragments which hybridized to the pBOS13 probe and thus are confirmed as transgenic plants.

Transgenic tobacco plants expressed the SLG gene at the RNA and protein levels as determined by RNA gel blot hybridization and binding of an anti-SLSG monoclonal antibody to protein gel blots. Expression of the SLG gene is specific to the stigma and style tissue in flowers and buds of transgenic plants.

Independent transformed plants expressed the SLG gene at different levels, with the highest levels of SLSG expression correlated to multiple sites of insertion of the SLG gene in two transgenic plants. Rearrangement of the introduced SLG gene apparently resulted in a higher level of SLSG in one transgenic plant and a lower level of expression in another. These differences in expression may result from differences in the site of insertion of the rearranged DNA in the N. tabacum genome and/or differences in sequence orientation within the rearranged SLG gene.

The anti-SLSG antibody recognized proteins of transgenic tobacco similar in electrophoretic mobility to the B. oleracea S13 SLSG. In B. oleracea the three molecular weight forms of SLSG are suggested to derive from different posttranslational modifications of the same primary translational product. The results obtained in transgenic tobacco demonstrate that the three forms are derived from a single SLG gene, and that the stylar tissue of tobacco correctly processes the three forms as in B. oleracea.

Maximal levels of SLSG occurred in open flowers of transgenic tobacco, consistent with expression of the self-incompatibility phenotype in *B. oleracea* and *N. alata*, and with reported expression of S-proteins in both systems. Expression of the SLG gene in transgenic tobacco differed from *B. oleracea* in that maximal levels of transcript occurred in open flowers in transgenic plants ; in *B. oleracea* maximal transcript is observed one day before flowering and decreases at anthesis. SLG transcript in stigma and style of transgenic plants is present in the earliest bud stage examined, 5 days before flowering, however, protein blots did not show detectable SLSG until 3 days before flowering. A similar pattern of protein *vs* transcript accumulation is shown in self-incompatible *N. alata*. In *N. alata*, S-associated proteins are not detected at the immature bud or "green bud" stage but are present in styles of mature buds ; the transcript of the cloned S-associated gene of *N. alata* is, however, present at the green bud stage.

The SLG gene is expressed in the stylar transmitting tissue of transgenic tobacco, as demonstrated by silver-enhanced immunogold labeling, consistent with S-associated transcript localization in *N. alata*. The transmitting tissue is the site where pollen tubes of *N. alata* and other Solanaceous self-incompatible species encounter a self-incompatibility barrier. In the immunocytochemical analysis of the stigma and style of transgenic *N. tabacum*, only very low levels of expression of SLSG are detected in the stigmatic epidermis and papillar cells as compared with high levels of SLSG in transmitting tissue is found.

*N. tabacum* transformed with the *B. oleracea* SLG gene expressed the gene in a manner more analogous to the gametophytic self-incompatibility system represented by *N. alata* than to the sporophytic *B. oleracea* system, where the SLG gene is expressed only in the stigmatic papillar cells. This represents a new level of homology between the two different self-incompatibility systems. The protein-coding regions of the SLG genes of *B. oleracea* and the S-associated genes of *N. alata*, as previously noted, do not share significant sequence similarity. Possibly the promoter regions of the *B. oleracea* and *N. alata* self-incompatibility genes share sequence motifs which interact with different spatial and temporal regulatory systems presented in the host plants. The different organization of the stigma in the two species (dense papillar cells and "dry" stigma in *Brassica*, sparse papillar cells and "wet" glandular stigma in *Nicotiana*) and the different physiology of pollen tube growth in a self-incompatible response may reflect genereally different gene regulatory systems in the two species. In some way which is not understood the host plant *N. tabacum* is able to express a gene for *Brassica* self-incompatibility in the tissues appropriate to a self-incompatibility response in that host.

Interestingly, the transgenic plants are not self-incompatible as determined by seed set although the SLG gene is expressed. The full complement of genes necessary to effect self-incompatibility may not be present in transgenic tobacco. S-related genes which do not show linkage to the S-locus are expressed in *Brassica* [Lalonde et al., 1989]. These genes and genes unrelated to the S-multigene family may be essential to the operation of self-incompatibility in *Brassica*. It is also possible that the molecular structure of *B. oleracea* SLSG, expressed at the dry stigma surface in *Brassica*, is not suitable for inhibition of pollen tubes at advanced stages of growth and/or cannot inhibit pollen tube growth when expressed in the glandular matrix of the *N. tabacum* style. Results of tobacco transformation experiments using the *N. alata* S-associated genes have not yet been reported but are of interest especially if the *N. alata* S-coding region is more effective than that of *B. oleracea* at influencing compatibility in *N. tabacum*. Transformation of *Brassica* with *N. alata* S-associated genes, when available, would allow further comparison between the sites of expression of sporophytic and gametophytic self-incompatibility genes.

As discussed above, we have been successful in introducing cloned $S_{13}$ and $S_8$ alleles of the SLG gene into *B. napus*. In addition, both cloned $S_{13}$ and $S_{22}$ alleles have been successfully introduced into *Nicotiana*, and S alleles have been introduced into *Arabidopsis* as well.

## Example VIII : Single cross hybrid production

Plants from a self-compatible, herbicide resistant line of plants that are heterozygous for the gene code for an S-locus specific glycoprotein linked with a gene coding for herbicide resistance (S⁺S⁻) (Line "A") are crossed with a self-fertile, herbicide sensitive line of plants (S⁻S⁻) (Line "B"). This cross produces an F1 hybrid generation that is 50 % self-incompatible, herbicide resistant (S⁺S⁻) and 50 % self-fertile, herbicide sensitive. Pollen from the self-fertile, herbicide sensitive plants will fertilize both self-fertile, herbicide sensitive and self-incompatible, herbicide resistant plants. Thus the hybrid crop will be fully pollinated and will have normal seed set.

Of course, the self-incompatible plants used in the present invention may contain more than one self-incompatible allele linked together.

## Example IX : Production of hybrid seed in the field

Plants from Line A (self-incompatible, heterozygous for the gene coding for an S-locus specific glycoprotein linked with the herbicide resistant gene) and Line B (self-fertile, herbicide sensitive) from Example VII are planted in separate strips. The male Line B is subsequently destroyed by application of the herbicide to which Line A is resistant before harvest. In this instance, all F1 hybrid seed will be derived from harvesting Line A.

Alternatively, Lines A and B may be interplanted and Line B destroyed by subsequent application of herbicide between pollen production and harvest of the seed. Again, all F1 hybrid seed will be derived from harvesting Line A.

### Example X : Maintenance of the heterozygous line

To obtain and maintain the heterozygous self-incompatible, transformed herbicide resistant line of plants described in Example VII, self-incompatible, herbicide resistant plants, either homozygous $(S^+S^+)$ or heterozygous $(S^+S^-)$ for the self-incompatibility/herbicidal resistant traits, are crossed with self-fertile, herbicide sensitive $(S^-S^-)$ plants. The resulting progeny will be a mixture of self-incompatible, herbicide resistant plants $(S^+S^-)$ and self-fertile, herbicide sensitive plants $(S^-S^-)$.

After germination of the seed from the cross, a herbicide (corresponding to the herbicide to which the self-incompatible are resistent) is applied. The self-fertile, herbicide sensitive plants will be killed, therefore leaving only the heterozygous self-incompatible, herbicide resistant plants $(S^+S^-)$ alive.

### Example XI : Single cross hybrid production from lines containing different S-incompatible loci

Line "X" is used to designate a line of plants which are heterozygous, self-incompatible, herbicide resistant $(S'^+S^-)$, and in which the self-incompatibility and herbicide resistant genes are linked. Line "Y" is used to designate a line of plantes which are heterozygous, self-incompatible, herbicide resistant $(S''^+ S^-)$, and in which the self-incompatibility and herbicide resitance are linked. S' and S'' are genes which code for different S-locus specific glycoproteins. The progeny of these lines will comprise mixtures of hybrids containing plants in equal proportions with the following self-incompatibility alleles : $(S'^+S''^+)$, $(S'^+S^-)$, $(S''^+S^-)$ and $(S^-S^-)$. The $(S'^+S''^+)$ progeny will be self-incompatible but with the capability of being fertilized by pollen from the $(S^-S^-)$ plants. The Line X and Line Y progeny (having the same genes as the parent) are self-incompatible, but cross-fertile with each other as well as capable of being fertilized by the $(S^-S^-)$ plants. The $(S^-S^-)$ progeny are self-compatible, capable of being fertilized by other like plants, and herbicide sensitive.

### Example XII : Maintenance of lines with different S-incompatible loci

Heterozygous self-incompatible, herbicide resistant Lines X and Y may be maintained in the same manner as Line A is maintained in Example IX.

### Example XIII :

Plants of line X and Y may be interplanted because they are both self-incompatible. It is not necessary to effectively separate the parent lines, as in Example VIII. Also, no herbicide application is necessary.

### Example XIV : Construction of plasmids pMTK8 and pMTK17

An SLG gene promoter fragment is isolated from the SLG-13 gene [Nasrallah JB and Nasrallah ME (1988)]. The oligonucleotid 5'-GAAAAAGGAGGGGA<u>TCC</u>AAAT<u>G</u>AAAGGCG-3'is used to introduce three nucleotide changes (underlined) in the SLG-13 gene by site-directed mutagenesis [Kunkel TA (1985)]. This mutation generated a BamHI site 8 bp upstream of the SLG-13 ATG codon. The $S_{13}$ promoter fragment extended from this introduced BamHI site to a natural BamHI site 3.65 kb upstream of the SLG-13 ATG codon.

The 3.65 kb BamHI $S_{13}$ promoter fragment is fused to the 280 bp SstI-EcoRI fragment isolated from plasmid pBI121 [Jefferson RA et al (1987)] that contains the polyadenylation signal from the nopaline synthase gene of the Agrobacterium tumefaciens Ti plasmid [Bevan MW et al (1983a) ; Bevan MW et al (1983b)], and these sequences are inserted into the binary vector pBIN19 [Bevan MW (1984)]. A 795 bp BglII fragment that encodes the diphtheria toxin A polypeptide is isolated from plasmid DT-A [Palmiter RD et al (1987)] and inserted into a BamHI site between the S-$_{13}$ promoter and the nopaline synthase polyadenylation signal. The resulting plasmid pMKT17, has a kanamycin resistance gene for transformation selection and the $S_{13}$ :DT-A fusion within the T-DNA borders.

An $S_{13}$ :GUS fusion is made by inserting the 3.65 kb BamHI $S_{13}$ promoter fragment into the BamHI site upstream of the GUS gene in the binary vector pBI101 [Jefferson RA et al (1987)]. The resulting plasmid, pMKT8, has a anamycin resistance gene for transformation selection and the $S_{13}$ :GUS fusion within the T-DNA borders.

## Example XV Tissue-specific expression of the GUS reporter gene

The resulting vector plasmid, pMKT8 is introduced into *Arabidopsis thaliana*, a member of the *Brassicaceae* that has a short generation time, is easily transformed, and in which the self incompatibility trait is not known to occur. However, the rapid flowering of regenerated transgenic plants makes *Arabidopsis* well suited for the analysis of flower-specific promoters such as those of the S-locus glycoprotein gene. Root segments are inoculated with *Agrobacterium* cells harboring pMKT8, and transgenic plants are seleced based on kanamycin resistance [transgenic plants are produced following infection of *Arabidopsis thaliana* (L.) Heynh. strain C24 root explants with *Agrobacterium tumefaciens* as described in PNAS USA 85 :5536 (1988) ; the *Agrobacterium* stain is pCIB542/A136, a strain derived from helper plasmid pEHA101 containing binary vector pMKT8 ; transformants are selected, and seeds produced by these plants are germinated on medium containing 50 mg/l kanamycin].

Starting with 125 root segments, 20 independent transgenic plants are obtained. Of these, 13 plants exhibited GUS activity in the stigma when assayed by histochemical staining with 5-bromo-4-chloro-3-indolyl glucuronide (X-Gluc) [see R.A. Jefferson *et al.* (1987). This assay involved introducing a solution containing 2 mM X-Gluc, 0.1 M sodium phosphate buffer at pH 7.0 and 0.5 % Triton X-100 into various plant tissues by a brief vacuum infiltration. After an overnight incubation at 37°C, the tissues are rinsed in 70 % ethanol and mounted for microscopic observation. A blue staining of the tissues is indicative of GUS activity.

Because the GUS assay is destructive, the flowers of primary transformants ($T_1$ generation) are saved to ensure seed production. Further studies are carried out on the kanamycin-resistant selfed progenies ($T_2$ generation) derived from 11 independent GUS-positive transformants.

Seed from the primary transformant plants are plated on kanamycin-containing medium and the number of green kanamycin-resistant ($km^r$) and bleached kanamycin-sensitive ($km^s$) seedlings is determined and tabulated in the following Table.

Segregation analysis and GUS activity in the $T_2$ progenies of transgenic *Arabidopsis*.

| $T_1$ plants | $T_2$ plants | | GUS activity | |
|---|---|---|---|---|
| | $Km^r$ | $Km^s$ | stigma | anther |
| 1 | 48 | $19^a$ | +++ | - |
| 2 | 89 | $35^a$ | ++++ | ++ |
| 3 | 60 | $7^b$ | +++ | - |
| 4 | 44 | $18^a$ | ++++ | ++ |
| 5 | 13 | 62 | ++++ | + |
| 7 | 126 | $39^a$ | +++ | - |
| 13 | 33 | $10^a$ | +++ | - |
| 16 | 58 | $21^a$ | +++ | - |
| 18 | 108 | $28^a$ | +++ | - |
| 20 | 25 | $14^a$ | ++++ | ++ |
| 23 | 35 | $13^a$ | +++ | - |

[a] and [b] indicate a 3:1 and 15:1 ratios, respectively (significant at the 5 % level based on $X^2$ tests).

(-) indicates no GUS activity; (+) indicates GUS activity; the number of (+) symbols indicate differences in intensity of GUS staining.

For ten transformants, the observed km$^r$ :km$^s$ segregation ratios are either 3 :1 or 15 :1. Thus indicating that these plants carried one or two T-DNA integrations, respectively. The ratio for transformant 5 is aberrant, and could not be used to predict the number of T-DNA integrations. The intergration of transforming DNA in several km$^r$ T$_2$ plants is additionally analyzed by DNA blot hybridization to physically characterize the T-DNA insert in GUS positive transformants. A restriction fragment of the expected 3.65 kb size is idenfied when the 3.65 kb BamHI S-locus glycoprotein gene promoter fragment is used to probe BamHI-digested DNA isolated from various transformants. All transformants contained the predicted 3.65 kb S$_{13}$ promoter fragment ; some contained additional bands homologous to the S-promoter probe, suggesting that they contained rearranged T-DNA copies. The pattern of hybridization with the GUS probe indicated that all transformants contained multiple T-DNA copies ; in some transformants these copies may have been genetically linked, or some reporter gene fusions may have been inactive as indicated by the segregation of km$^r$ found in selfed progeny. No hybridization is observed with DNA obtained from a control untransformed Arabidopsis plant.

All kanamycin-resistant T$_2$ plants exhibited GUS activity in the pistil. The blue GUS staining is detected in the papillar cells of the stigma surface, is not detected in style or ovary, and increased as the stigma matured. The highest intensity of staining is observed in the progeny from four transformants, specifically plants 2, 4, 5 and 20. In these plants, GUS activity is detected in anthers as well as in stigmas as depicted in te preceding Table. The intensity of staining of anthers varied, and is strongest in the progenies of plants 2, 4 and 20, and weakest in the progeny of plant 5. In the progenies of the remaining 7 transformants, the fusion gene is either not expressed in the anthers or is expressed at a level too low to be detectable under the conditions of the histochemical assay. The variation in different transgenic lines is attributed to effects of position of integration.

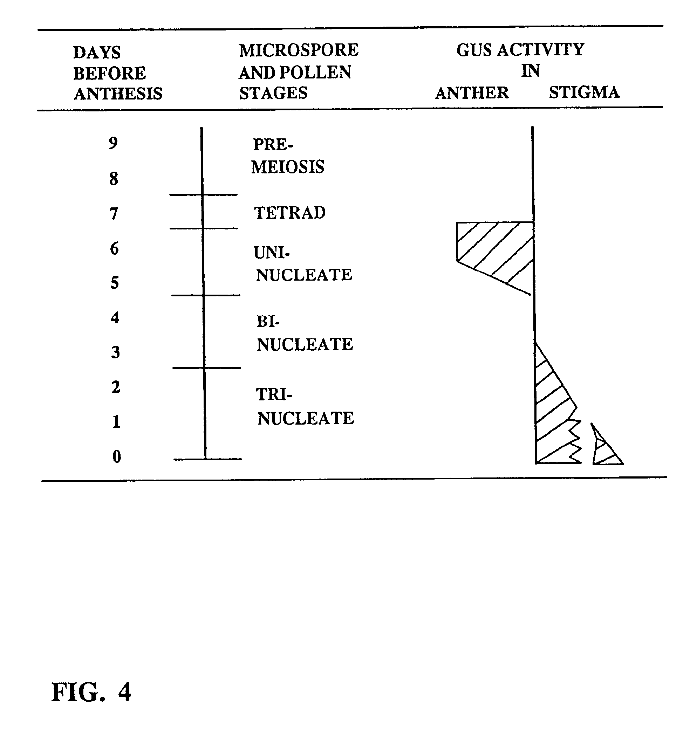

The development pattern of S-locus glycoprotein gene promoter activity is also analyzed in progeny plants of transformants 2, 4, 5 and 20. No GUS activity is detected in the leaves, stems, or roots of the transgenic seedling plants. In all transgenic lines, only the stigmas and anthers showed GUS activity. A similar spatial and temporal pattern of reporter gene expression is observed in these lines, and is inherited in the T$_3$ generation. This pattern is summarized diagrammatically in Fig. 4. More specifically, this figure is a summary of the histochemical detection of GUS activity in the anther and stigma tissues of transgenic Arabidopsis as a function of microspore and pollen development. In this figure, "days before anthesis" refers to the age of flower buds ; the cross-hatched areas are estimates of the relative staining intensities and are intended to depict the general developmental pattern of expression in diagrammatic form.

As depicted in Figure 4, GUS activity is absent in premeiotic buds and buds of the tetrad stage at 7 days before anthesis. At the early uninucleate microspore stage at 6 days before anthesis, blue staining is apparent in cells of the tapetum, in correlation with the beginning of exine deposition. Neither anthers incubated in the GUS assay buffer without X-Gluc nor anthers of untransformed control plants showed any blue staining, excluding the possibilities of non-specific staining and intrinsic GUS activity. No staining is detected in microspores or mature pollen grains. The intensity of staining in the tapetum is highest at the early uninucleate stage and gradually decreased in buds at 5 days before anthesis which correspond to the late uninucleate microspore stage. Staining disappeared in buds containing binucleate pollen, coincident with tapetal cell degeneration at 4 days before anthesis. At this stage, neither anther nor stigma showed any GUS activity. In subsequent developmental stages, no GUS activity is detected in anther tissue, in isolated mature pollen grains, or in pollen after germination on the stigmas of untransformed plants. However, in buds at 3 days before anthesis (corresponding to the late binucleate pollen stage) faint blue staining could be detected in the stigmatic papillae. Staining of the stigma is clearly visible in buds containing trinucleate pollen at 2 days before anthesis, and is most intense in the open flower. The intensity of GUS staining of the mature stigma is much stronger than that observed in the tapetum of the younger anther.

The temporal regulation of fusion gene expression is further characterized by isolating microspores from buds at different developmental stages. These are double-stained, first with X-Glu to localize GUS activity, and then with the DNA fluorochrome DAPI (4'-6-di-amidino-2-phenylindole) which is used at a concentration of 1 μg/ml [see T. Kuroiwa et al. (1982)] in a buffer containing 20 mM Tris-HCl at pH 7.6, 0.25 M sucrose, 1 mM EDTA, 1 mM MgCl$_2$, 0.1 mM ZnSO$_4$, 0.1 mM CaCl$_2$, 0.8 mM phenylmethylsulfonylfluoride, 0.05 % β-mercaptoethanol, and 1 % Triton X-100. The DAPI is used to identify the nuclear condition of the microspore. GUS staining is observed primarily in binucleate pollen grains, although weak staining is occasionally observed in mononucleate microspores. Staining of microspores is not detected until after the initiaon of tapetal cell degradation. GUS activity is not detected in the tapetum or in any other tissue of the anther.

The incidence of GUS staining in pollen of mature flowers is determined by counting the number of grains that are stained blue after incubation with X-Glu. Two plants predicted to have an active T-DNA at one genetic locus showed blue staining in 50 % of its pollen grains, and one plant predicted to have an active T-DNA at two loci showed blue staining in 75 % of its pollen grains. This incidence of staining is consistent with the observation of gametophytic expression in the haploid pollen grain, rather than sporophytic expression by some other tissue of the anther.

These data resulting from the expression of this reporter gene function have demonstrated that the specific expression of a single S-locus glycoprotein gene promoter can function in two tissue types found in the flower which are involved in the self incompatibility reaction : the papillar cells of the stigma surface and the tapetal cells of the anther. Expression of the S-locus glycoprotein gene promoter is not coordinately regulated in the two cell types, but is temporally separated by a period of approximately 3 days in the development of the flower. This data suggests that a single protein determinant may direct the self incompatibility reaction in these two tissues.

In the stigma, the site and timing of expression coincided with the previously described expression of S-locus glycoprotein genes in *Brassica*. *Arabidopsis* is self-fertile genus with no known self-incompatible strains. Nevertheless, the drastic increase in GUS expression in stigmas at one day before anthesis paralleled the acquisition of the self incompatibility response by the developing stigma in self-incompatible *Brassica* strains. In the anther, GUS expression is observed in the tapetum during a narrow developmental window after meiosis. This result implies that sporophytic control of self incompatibility phenotype in crucifers is based on the expression of the S-locus postmeiotically in the tapetum, rather than on premeiotic expression as has been previously suggested by K.K. Pandy (1960). In comparison with mature stigmas, anthers exhibited only weak staining however. This low level of expression may account for the fact that detection of the S-locus glycoprotein gene product in anthers of self-incompatible *Brassica* stains has met with little success to date. However, as shown in the present invention, the single S-locus glycoprotein gene has the requisite expression profile for the determination of the pollen-stigma interaction of incompatibility ; the novel findig of S-locus glycoprotein promoter activity in the anther supports the hypothesis that sporophytic control of self incompatibility is a result of s-locus glycoprotein gene expression in the tapetum.

## Example XVI Tissue specific expression of a transformed toxin gene

In addition to the reporter gene fusion described above, a second fusion gene construct is made in order to further characterize the sites of S-gene expression as well as to study the function of tissues that express the S-gene. In this construct, the 3.65 kb $S_{13}$ gene promoter fragment is fused to the coding sequence of the Diphtheria toxin A polypeptide in the plant transformation vector pBIN19 [see M Bevan (1984)] to generate plasmid pMKT17. Diphtheria toxin A polypeptide is known to block protein synthesis through ADP-ribosylation of elongation factor II in many organisms, including plants [see R.J. Collier (1977)], and has also been demonstrated to have nuclease activity [see M.P. Chang *et al.* (1989)] These cytotoxic effects allow a single molecule of Diphtheria toxin A polypeptide to cause cell death [see M. Yamizumi *et al.* (1978)], and so Diphtheria toxin A polypeptide can serve as a very sensitive indicator of gene expression. Gene fusions to Diphtheria toxin A polypeptide have been used to geneticaly direct cell death in a variety of mammalian tissues [see R.R. Behringer *et al.* (1988)].

The $S_{13}$ :Diphtheria toxin A polypeptide toxic gene fusion is introduced into tobacco by *Agrobacterium*-mediated transformation. Eight independent kanamycin resistant (km$^r$) transformants are obtained and characterized together with eight untransformed control plants. Drastic morphological differences are observed between flowers, but not other tissues, of untransformed controls and six of the eight $S_{13}$ :Diphtheria toxin A polypeptide transformants. The six positive transformants could be divided into two classes based on the severity of the phenotype induced by toxic gene expression. In five Class I transformants, the effects of toxic gene expression are relatively moderate, while in one Class II transformant they are more severe. The differences in phenotype between Class I and Class II transformants is attributed to differences in the level of toxic gene function expression.

As for the $S_{13}$ :GUS fusion described earlier, the effects of $S_{13}$ :Diphtheria toxin A polypeptide fusion expression are apparent in two tissues of the flower, the pistil and the pollen. In Class I transformants, flowers appeared normal externally, and typically reached maturity. The styles of these flowers are, however, dramatically shorter than those of the control plants. Style length in open flowers of these transformants ranged from 4 to 9 mm, as compared with 34 to 36 mm in control plants. At the surface of the style, the stigma, there are additional morphological differences between the transformants and controls. The secretory zone of the stigma is ablated in transformants, causing a reduction in size of the stigma surface, and a change in the appearance of this surface tissue. Stigmas of transformants are white, and often contained regions of brown necrotic tissue, while those of control plants are bright green. Additionally, some stigmas of some transformants contained three rather than two lobes. This tri-lobed structure is found throughout the pistil, in the stigma, style and ovary.

A more detailed analysis of the stigma structure is conducted by scanning electron microscopy. As seen when the stigma surface of buds at different developmental stages is analyzed, the effects of toxic gene expression increased as buds aged. Buds at 5 days prior to flowering are nearly normal ; the stigma surface had only a slight central indentation which became more severe in older buds, in addition there is a deep furrow in mature buds at 1 day prior to flowering. This is in dramatic contrast to the rounded stigma surface of the untransformed control. The papillar cells at the stigma surface are present in the toxic fusion gene transformants, however the morphology of some papillae is abnormal.

Microscopic analysis of thin sections through the style is used to identify the cell types affected by toxic gene expression. In untransformed tobacco, the transmitting tissue filled the central area of the style. Expression of the $S_{13}$ :Diphtheria toxin A polypeptide fusion resulted in ablation of the transmitting tissue of the style. In mature buds, the transmitting tissue in the region of the style near the stigma is compressed, and ultrastructural analysis showed that cells in this region are dead. In the region of the style near the ovary, the transmitting tissue is completely ablated, and hollow due to the death of the transmitting tissue. The outer parenchymal tissue of the style is unaffected by toxic gene expression. The tissue of the ovary appeared normal when analyzed by light microscopy.

Pollen from untransformed control plants is used to pollinate transformants, and the growth of pollen tubes is analyzed microscopically after staining with aniline blue. Pollen did not germinate on the ablated stigmas of open flowers. Additionally, transformants never set seed through either self-pollination or hand pollination with control pollen, further indicating that ablated pistils are unable to support pollen tube growth. Pollen germination is also analyzed in young buds in which the pistil had only minor morphological differences from untransformed controls. Although pollen did germinate on the stigmas of young buds, pollen tubes are never observed growing.

The buds of Class II transformant are often withered due to abscissions from the flowering stem. Abscission occurred before buds matured, typically by three days prior to flowering, and open flowers are never observed on this plant. The effects of Diphtheria toxin A polypeptide expression on the pistil are more severe than in Class I transformants. Notably, the style is very short (1-2 mm), and is not morphologically distinct from the ovary. Additionally, fusion of the carpels is incomplete, and the morphology of the stigma is bizarre. Ovary structure is also altered in this transformant : the placenta is smaller than in controls, the ovules are dead or absent, and there are large open spaces in the locules.

Pollen is also affected by expression of Diphtheria toxin A polypeptide as characterized in Class I transformant. Pollen from open flowers of toxic gene transformants is a mix of normal round grains and small collapsed grains. In transformants, greater than 50 % of the pollen grains are small and collapsed, while in untransformed controls less than 15 % had this morphology. When pollen from toxic gene transformants is germinated in vitro, only round grains germinated, indicating that the collapsed grains are not viable.

DNA blot hybridization analysis is used to physically characterize the T-DNA insert in Diphtheria toxin A polypeptide transformants. An $S_{13}$ promoter probe hybridized to the predicted 3.65 kb $S_{13}$ promoter fragment in BamHI-digested DNA of all transformants. The intensity of hybridization to this probe varied in different transformants, and in particular is strongest in the Class II transformant. The severe phenotype in this transformant may have been the result of multiple T-DNA insertions. Alternatively, the site of T-DNA integration may have affected the level of toxic gene expression.

Thus, as described above, the tissues are affected by toxic gene expression. In the tobacco pistil, the Brassica S-gene promoter is expressed in the secretory zone of the stigma, in the transmitting tissue of the style, and in the placenta of the ovary. S-gene expression is also observed in pollen of transgenic tobacco. Although previous attempts to identify the S-locus glycoprotein gene in pollen of Brassica and transgenic tobacco are unsuccessful, the sensitivity of the GUS histochemical assay and the Diphtheria toxin A polypeptide bioassay allowed detection of these fusion gene products. The results of the present invention clearly indicate that a single S-locus gene may actually direct the incompatibility response in pistil and pollen.

Thus while we have illustrated and described the preferred embodiment of our invention, it is to be understood that this invention is capable of variation and modification and we therefore do not wish to be limited to the precise terms set forth, but desire to avail ourselves of such changes and alternations which may be made for adapting the invention to various usages and conditions. Accordingly, such changes and alterations are properly intended to be within the full range of equivalents, and therefore within the purview of the following claims.

Having thus described our invention and the manner and a process of making and using it in such full, clear, concise and exact terms so as to enable any person skilled in the art to which it pertains, or with which it is most nearly connected, to make and use the same.

## BIBLIOGRAPHY

**Bateman AJ**, Heredity 9 : 53 (1955)
**Behringer RR** et al., Genes Dev. 2 : 453 (1988)
**M Bevan**, Nucleic Acids Res. 12 : 8711 (1984)

Bevan MW *et al*, Nucl. Acids Res. 11, 369-385 (1983a)

Bevan MW *et al*, Nature(London) 304, 184-187 (1983b)

Bingham PM *et al*, Cell 25, 693-704 (1981)

Cashmore A, "Genetic Engineering of Plants", An Agricultural Perspective, Plenum, New York (1983) ; pp 29-38].

Chang et al., Science 246 : 1165 (1989)

Collier RJ, "The specificity and action of animal, bacterial and plant toxins", P. Cuatrecasas, Ed. Chapman and Hall, London (1977)

Dwyer KG *et al.*, Genome 31 : 969 (1989)

Frank G *et al*, Cell, 21 : 285-294 (1980)

Fromm *et al*, Proc. Natl. Acad. Sci., USA 82 : 5824 (1985)

Fry *et al*, " Transformation of Brassica napus with Agrobacterium tumefaciens based vectors", Plant Cell Rep. 6 : 321 (1987)

Gardner RC *et al*, Nucl. Acids Res., 9 : 2871-2888 (1981)

Grimsley NH *et al*, Nature 324 : 177-179 (1987)

Heslop-Harrison J, Nature 218 : 90 (1968)

Heslop-Harrison J, Ann. Rev. Plant Physiol. 26 : 403 (1975)

Hollingshead, S. and Vapnek, D., Plasmid 13 : 17-30 (1985)

Hohn T *et al*, in : "Molecular Biology of Plant Tumors", Academic Press, New York, pp. 549-560 (1982)

Hood, EE *et al*, Bio/Technology 2 : 702-708 (1984)

Hood, EE *et al*, J. Bacteriol. 168 : 1291-1301 (1986)

Horsch RB *et al*, Science 227 : 1229-1231 (1985)

Howard *et al*, Planta 170 : 535 (1987)

Jacoby, G *et al*, J. Bacteriol. 127 : 1278-1285 (1976)

Jefferson RA *et al*, EMBO J. 6 : 3901-3907 (1987)

Kandasamy MK *et al*, Dev. Biol. 134 : 462 (1989)

Kunkel TA, Proc. Natl. Acad. Sci., USA 82, 488-492 (1985)

Kuroiwa T, *et al*, Nature 298 : 481 (1982)

Lalonde *et al*, Plant Cell 1 : 249-258 (1989)

Mackay GR, Euphytica 26 : 511 (1977)

Maniatis *et al*, "Molecular Cloning", A Laboratory Manual, Cold Spring Harbor Laboratories, 1982

Matzke, AJM & Chilton M-D, J. Mol. Appl. Genet. 1 : 39-49 (1981)

Moore HM and Nasrallah JB, The Plant Cell 2, 29-38 (1990)

Morelli *et al*, Nature 315 : 200 (1985)

Nasrallah JB *et al*, Ann. Rev. Genetics 23 : 121 (1989)

Nasrallah JB *et al*, Proc. Natl. Acad. Sci, USA 85 : 5551 (1988)

Negrutiu I *et al*, Plant Mol. Biol., 8 : 363-373 (1987)

Neuhaus *et al*, Theor. Appl. Genet., 74 : 30 -36 (1987)

Palmiter RD *et al*, Cell 50, 435-443 (1987)

Pandy KK, Evolution 14 : 98 (1960)

Petit *et al*, Mol. Gen. Genet., 202 : 388 (1986)

Potrykus I and Shillito RD, Methods in Enzymology. Vol 118, Plant Molecular Biology, eds. A and H Weissbach, Academic Press, Orlando, 1986

Rogers SG *et al*, "Use of cointegrating Ti plasmid vectors.", in : Gelvin SB, Shilperoort RA (eds.). Plant Mol. Biol. Manual, Kluwer Academic Publishers (1988)

Rothstein *et al*, Gene 53, 153-161 (1987)

Schocher RJ *et al*, Bio/Technology, 4 : 1093-1096 (1986)

Sciaky, D, Montoya, AL & Chilton, M-D (1978)

Shillito *et al*, Bio Technology, 3 : 1099-1103 (1985)

Southern EM, J. Mol. Biol. 98 : 503-517 (1975)

Towbin H *et al*, Proc. Natl. Acad. Sci., USA 76 : 4350-4354 (1975)

Spena *et al*, EMBO J. 4 : 2736 (1985)

Valvekens D *et al*, Proc. Natl. Acad. Sci., USA 85 : 5536-5540 (1988)

Van Sluys *et al*, "Studies on the introduction and mobility of the maize activator element in Arabidopsis thaliana and Daucus carota", EMBO J. 6 : 3881 (1987)

Wang Y-C *et al*, Plant Mol. Biol., 11 : 433-439 (1988)

Yamizumi M *et al.*, Cell 15 :245 (1978)

## SEQUENCE LISTING

**SEQ ID NO : 1**
**SEQUENCE TYPE :** Nucleotide with corresponding protein
**SEQUENCE LENGTH :** 2236 bases
**STRANDEDNESS :** single
**TOPOLOGY :** linear
**MOLECULAR TYPE :** genomic DNA
**ORIGINAL SOURCE**
**ORGANISM :** *Brassica oleracea var. acephala*
**PROPERTIES :** SLG-13 self-incompatibility gene

```
                                                          50
GAGCTCTCTG TGCTATTAGA ACATTTATCG TTCATAACCT GACAAATCAA

                                                         100
ACGTCCTAAA ACAATACAAA TTTTCATTTT GCAGAACACC AAACGAGTTG

                                                         150
TTCGAGATAG TCCCATCATA GGTATAACTT AAAGATTGAC GAATAGGAGT

                                                         200
TGAGAAACTG GAAACAAGGG CTTGAGTTAC GATATATCGG AGCGGCTGGG

                                                         250
AGCATGTCAC TCGATTTGGC TTGTTTATAG GTTGATTGAT TAAATAAAAC

                                                         300
AAAAGCATAT AGTTCGCCGG CCCGGTTGTT GCAACCTTCC AAGGAACGCC

                                                         350
TGCTGAGATG GATTTACAAT TTGATTTCTT TTGTATTTTT ATTTGGTGTG

                                                         400
TTTAATATAT TAGTTAACCA ATTTACGTTA TACCAAATTT TTCAACCCTC

                                                         450
TTTTTAGTAA AAAACGAAAT TAAAGTTTTT TCCCTCTTAG TCCGACGATT

                                                         500
TTAAGCTAAT TAGTTCGAAC AAAGAGTACA ACATTAATTT TCTAACAGAC

                                                         550
TTAGATGCAC TTGCGAACAA CATACTTGCT GAACACCATA TGTTATGTTG
```

28

```
                                                           600
GCAGGGTGAG AAATTAATCA CGTGTAGATA TAGAAGTAGT AGACAAATGA

                                                           650
TATAGGTTTG TGGGAATGAA TTAATCGATG GGATGAAAAA GTCATCGAAC

                                                           700
ATGTAACACC ACATTTTACT TGTCTGCTAG GTTCGTGATA GTCGTTTAAA

                                                           750
TTAGATACGT GAAAAAAGAT TATAAATATG CAAAAGGGGA AGGGGAAGAA

AAGAAAGAAA AAGGAGGGGA
                                                  800
                                                                    GA
GAA ATG AAA GGC GTT AAA AAA ACC TAC GAT ATT TCT TAC ACT TTA TCC
Glu Met Lys Gly Val Lys Lys Thr Tyr Asp Ile Ser Tyr Thr Leu Ser

                                      850
TTT TTG CTT GTC TTT TTC GTC TTG ATT CTA TTT CGT CCT GCC TTT TCG
Phe Leu Leu Val Phe Phe Val Leu Ile Leu Phe Arg Pro Ala Phe Ser

                                      900
ATC AAC ACT TTG TCG TCT ACA GAA TCT CTT ACA ATC TCA AGC AAT AGA
Ile Asn Thr Leu Ser Ser Thr Glu Ser Leu Thr Ile Ser Ser Asn Arg

                                          950
ACA CTT GTA TCT CCC GGT AAT GTC TTC GAG CTC GGT TTC TTC AAG ACC
Thr Leu Val Ser Pro Gly Asn Val Phe Glu Leu Gly Phe Phe Lys Thr

                                              1000
ACA TCA AGT TCT CGT TGG TAT CTC GGG ATA TGG TAC AAG AAA TTT CCA
Thr Ser Ser Ser Arg Trp Tyr Leu Gly Ile Trp Tyr Lys Lys Phe Pro

                                                  1050
TAT AGA ACC TAT GTA TGG GTT GCC AAC AGA GAT AAC CCT CTC TCC AAT
Tyr Arg Thr Tyr Val Trp Val Ala Asn Arg Asp Asn Pro Leu Ser Asn

                                                  1100
GAC ATT GGA ACC CTT AAA ATC TCA GGC AAT AAT CTT GTC CTC CTT GAT
Asp Ile Gly Thr Leu Lys Ile Ser Gly Asn Asn Leu Val Leu Leu Asp

                                                  1150
CAC TCC AAT AAA TCT GTT TGG TCG ACG AAT GTG ACT AGA GGA AAT GAG
His Ser Asn Lys Ser Val Trp Ser Thr Asn Val Thr Arg Gly Asn Glu
```

```
                                                                    1200
AGA TCA CCG GTG GTG GCA GAG CTT CTC GAT AAT GGA AAC TTC GTG ATG
Arg Ser Pro Val Val Ala Glu Leu Leu Asp Asn Gly Asn Phe Val Met


                                                                    1250
CGA GAC TCC AAT AGC AAC AAC GCA AGT CAA TTC TTG TGG CAA AGT TTC
Arg Asp Ser Asn Ser Asn Asn Ala Ser Gly Phe Leu Trp Gln Ser Phe


                                                                    1300
GAT TAC CCT ACA GAT ACT TTG CTT CCA GAG ATG AAA CTG GGT TAC GAT
Asp Tyr Pro Thr Asp Thr Leu Leu Pro Glu Met Lys Leu Gly Tyr Asp


                                                                    13
CTC AAA ACA GGG TTG AAC AGG TTC CTT ACA TCA TGG AGA AGT TCA GAT
Leu Lys Thr Gly Leu Asn Arg Phe Leu Thr Ser Trp Arg Ser Ser Asp


50
GAT CCG TCA AGC GGG GAT TAC TCG TAC AAG CTC GAA CTC CGA AGA CTT
Asp Pro Ser Ser Gly Asp Tyr Ser Tyr Lys Leu Glu Leu Arg Arg Leu


   1400
CCC GAG TTT TAT CTA TCG AGT GGA TCT TTC CGA TTG CAT CGG AGT GGT
Pro Glu Phe Tyr Leu Ser Ser Gly Ser Phe Arg Leu His Arg Ser Gly


      1450
CCA TGG AAT GGA TTC CGA ATT AGT GGG ATA CCA GAG GAC CAA AAG TTG
Pro Trp Asn Gly Phe Arg Ile Ser Gly Ile Pro Glu Asp Gln Lys Leu


         1500
AGT TAC ATG GTG TAC AAT TTC ACA GAG AAT AGT GAA GAG GCC GCA TAT
Ser Tyr Met Val Tyr Asn Phe Thr Glu Asn Ser Glu Glu Ala Ala Tyr


         1550
ACA TTC CTA ATG ACC AAC AAC AGC TTC TAC TCG AGA TTG ACA ATA AGC
Thr Phe Leu Met Thr Asn Asn Ser Phe Tyr Ser Arg Leu Thr Ile Ser


            1600
TCC ACA GGG TAT TTT GAG CGA CTA ACG TGG GCT CCG TCA TCA GTG GTA
Ser Thr Gly Tyr Phe Glu Arg Leu Thr Trp Ala Pro Ser Ser Val Val


            1650
TGG AAC GTG TTC TGG TCT TCT CCT AAC CAC CAG TGT GAT ATG TAC AGG
Trp Asn Val Phe Trp Ser Ser Pro Asn His Gln Cys Asp Met Tyr Arg


            1700
ATG TGT GGG CCT TAC TCT TAC TGT GAC GTG AAC ACA TCA CCG GTG TGT
Met Cys Gly Pro Tyr Ser Tyr Cys Asp Val Asn Thr Ser Pro Val Cys
```

```
                        1750
AAC TGT ATA CAA GGG TTC AGA CCC AAG AAC CGG CAG CAG TGG GAT CTG
Asn Cys Ile Gln Gly Phe Arg Pro Lys Asn Arg Gln Gln Trp Asp Leu


                        1800
AGA ATC CCG ACA AGT GGG TGT ATA AGA AGG ACA CGG CTG AGC TGC AGT
Arg Ile Pro Thr Ser Gly Cys Ile Arg Arg Thr Arg Leu Ser Cys Ser


                        1850
GGA GAT GGT TTT ACC AGG ATG AAG AAT ATG AAG TTG CCA GAA ACT ACG
Gly Asp Gly Phe Thr Arg Met Lys Asn Met Lys Leu Pro Glu Thr Thr


                        1900
ATG GCG ATT GTC CAC CGA AGT ATT GGT TTG AAA GAA TGT GAG AAG AGG
Met Ala Ile Val His Arg Ser Ile Gly Leu Lys Glu Cys Glu Lys Arg


                        1950
TGC CTT AGC GAT TGT AAT TGT ACC GCA TTT GCA AAT GCG GAT ATC CGG
Cys Leu Ser Asp Cys Asn Cys Thr Ala Phe Ala Asn Ala Asp Ile Arg


                        2000
AAT CGT GGG ACG GGT TGT GTC ATT TGG ACC GGA GAG CTT GAA GAT ATC
Asn Arg Gly Thr Gly Cys Val Ile Trp Thr Gly Glu Leu Glu Asp Ile


                        2050
CGG ACG TAC TTT GCT GAC GGT CAA GAT CTT TAT GTC AGA TTG GCT GCT
Arg Thr Tyr Phe Ala Asp Gly Gln Asp Leu Tyr Val Arg Leu Ala Ala


                        2100
GCG GAC CTT GTT TAGCTGTTTC TCTTAAAATA
Ala Asp Leu Val


                                           2150
AAACACGGAT CCGACTATGA TATCGAGAAA TCCGGATATG TAACGCAACT


                                           2200
ATCTTAATAA GTCTACCCTA AATATATATC TGAATCAATA AATGATATAC


                                           2250
AGATCGTATC CAAATCCTTT TTAATCCTTT TTAATATATA TAAGAAAACA


                                           2300
TGCCATCGAA ANTACAAATT TGATTATAAT ATTTTGCCGA CACCTGGAAA


                                2336
TTTACTTTCT TCGAATTTAT TTGTATTTGA AAGGCT
```


**SEQ ID NO : 2**
**SEQUENCE TYPE :** Nucleotide
**SEQUENCE LENGTH :** 1317

**STRANDENESS :** single
**TOPOLOGY :** linear
**MOLECULAR TYPE :** cDNA

**ORIGINAL SOURCE**
**ORGANISM :** *Brassica oleracea*
**IMMEDIATE EXPERIMENTAL**

SOURCE
MAME OF CELL CLONE : λgt10
PROPERTIES : The sequence alignments and differences in the coding region among four S-locus alleles of *Brassica oleracea* are given.

```
S6   1 ATGA AAGGAGTAAG AAAACCCTAC GATAATTCTT ATACCTTATC
S13          C   T A      A           T         C T
S14                      AA        A             C T
S22          C             A       C G     C   GC  A

CTTTTTGCTT GTCTTTTTCG TCTTGATTCT ATTTTGTCCT GCCTTTTCGA
                                     C
                             C  CC               A
         C                 TA     A    CAC  C  T

TCAACACTTT GTCGTCTACA GAATCTCTTA GAATCTCAAG CAACAG
                                 C                T
                   T             C      C
               G                 CG

141    AACAC TTGTATCTCC AGGTAATAAC TTCGAACTCG GCTTCTTCCG
                  C       GT         G      T      AA
                  C       GT         G             A
                  C       GT                T      A
```

```
AACCAACTCA AGTTCTCGTT GGTATCTCGG GATATGGTAC AAGAAATTGC
G     CA                                              T
      C     G                                       G  T
                                                       T

TCGACAGAAC CTATGTATGG GTTGCCAACA GAGATAACCC ACTCT
CAT T                                       T
C                                           T
C A                                         T   T

281   CCAAT GCCATTGGAA CCCTCAAAAT CTCAGGCAAT AATCTTGTCC
             A              T
      G  T                           T                A
      C  T     C                   A   A             TA

TCCTTGGTCA CACCAATAAA TCTGTTTGGT CGACGAATCT TACTAAGGAA
      A          T                      G         G
      A          T T                              A
                 T            A         C       G     AG

ATGAGAGACT TCCGGTGTGT GCAGAGCTTC TCTCTAATGG AAAC 421
      TC A                            GA
      TC                  CG          G
      GTC                             G

TTCGTGATGC GAGACTCCAG TAACAACGAC CAAGTGAATA CTTGTGGCAA
                 A     G      A          C     T
                 A                          G   T
     T           A              CG     T  CG   T

AGTTTCGATT ACCCTACGGA TACTTTGCTT CCAGAGATGA AACTGGGTTA
                A
           T    A                                    A
                A                              G

CGACCTCAAA ACAGGGTTGA ACAGGTTCCT TACATCATG  561   GAGAA
                 C          C                    C
                 C              A

GTTCAGATGA TCCATCAAGC GGGGATTTCT CGTACAAGCT CGAAACCCGA
                 G              A                     CT
                 G              A          A          C T
           T                                       AG G

AGCCTTCCTG AGTTTTATCT ATGGCATGGG ATCTTTCCAA TGCATCGGAG
  A       C                      C AG     A TCT  C G T
  A            A                 C AG     G      T T   T
  G            AC                C AG            G G       T
```

```
TGGTCCATGG AATGGGTCCG ATTTAGTGGC ATAC 701     CAGAGGACCA
                T          A          G
                A                        C              T
C                        C  A                             T        G


AAAGCTGAGT TACATGGTGT ACAATTTCAC AGAGAATAGT GAAGAGGTCG
      T                                                      C
      T              T            ...... T TTCAC GGA  ATGAGA
                     T                                A      G


CTTATACATT CCGAATGACC AACAACAGCA TCTACTCGAG ATTGACACTA
  A              T                      T                  A
       A                                T              AT TG
                                   C                       G


AGTTCCGAAG GGTATTTTCA GCGACTTACG     841      TGGAATCCGT
   C   AC              G          A                  GC
   T  TC            A  G       A  AG
   T  TC            A  G          AG


CAATAGGGAT ATGGAACAGG TTCTGGTCTT CTCCAGTGGA CCCCCAGTGC
  TC    T G           GT                 T...A     A           T
  TC            G          T          G   T    GT    TT A
  T             G          GT              T      T    T A


GATACATACA TAATGTGCGG GCCTTACGCT TACTGTGGCG TGAACACATC
      TG       GG      T            T             A
T       G      G GC    T            T             T
     G G       G GC    T            T             A


ACCTGTTTGT AACTGTATCC AAGGGTTCAA           981  TCCCC
   G G              A            G                    A    A
 G GA                                                     T
   GA                                                     T


GGAATATACA GCAGTGGGAT CAGAGAGTCT GGGCAGGTGG GTGTATAAGG
A    CCGG                T      A C A  A                   A
C       G G                                 AA
C       G GG


AGGACGCGGC TTAGCTGCAG TGGAGATGGT TTTACAAGGA TGAAGAACAT
        A          G                          C              T
                G               C                        T
         A                 C          C T
```

```
GAAGCTGCCA GAAACTACGA TGGCGATTGT CGACC       1121 GCAGT
         T                          C                A

         T                       T       A          G

ATTGGTGTGA AAGAATGTGA GAAGAGGTGC CTTAGCGATT GTAATTGTAC
         T                          A    C
                                                   C

TGCTTTTGCA AATGCGGATA TCCGGAATGG TGGGACGGGT TGTGTGATTT
C    A                        C                C
C    G
C    G                                G

GGACCGGACG GCTTGACGAT ATGCGGAATT ACGTT       1261 GCT..
         GA          A    C    CG    T              ..
    T    GA     C    G              G    C       A GG
                                         C         GA

.......CAC GGTCAAGATC TTTATGTCAG ATTGGCTGTT GCTGACCTTG
.......G                                   C      G
CGCTACTG   A                              CA       A
T......                                   C      A

TT   1317
```

## Claims

1. A method of imparting self-incompatibility to recipient plants comprising :
(a) isolating a structural gene which encodes for the production of an S-locus specific glycoprotein from a self-incompatible donor plant ; and
(b) integrating said structural gene into the genome of a recipient plant that does not ordinarily produce said S-locus specific glycoprotein, wherein said recipient plant is self-incompatible.

2. A method according to claim 1 wherein the donor plant is a self-incompatible strain of *Brassica*.

3. A method according to claim 1 wherein the structural gene is selected from the group consisting of the SLG-13, SLG-14, SLG-22 gene of *Brassica oleracea* and the SLG-8 gene of *Brassica campestris*.

4. A method according to claim 1 wherein the recipient plant is from a different family of plants from the self-incompatible donor plant.

5. A method according to claim 1 wherein said structural gene is stably integrated into the genome of the recipient plant using a strain of *Agrobacterium tumefaciens* cells as the means for integration.

6. A method according to claim 1 wherein the structural gene comprises the DNA sequence as given in sequence listing 1.

7. A method according to claim 1 wherein structural gene which encodes for the production of an S-locus specific glycoprotein from a self-incompatible donor plant is operably linked with one or more further structural genes.

8. A method according to claim 7 wherein the said further sturctural gene is a reporter gene the expression of which in a transgenic cell is indicative of the incorporation of the linked first structural gene into the trangenic cell's genome.

9. A recombinant DNA molecule comprising a structural gene which codes for the production of an S-locus specific glycoprotein from a self-incompatible plant, selected from the group consisting of the SLG-13, SLG-14, SLG-22 genes of *Brassica oleracea* and the SLG-8 gene of *Brassica campestris*.

10. A recombinant DNA molecule according to claim 9 wherein the structural gene which codes for the production of an S-locus specific glycoprotein comprises the DNA sequence as given in sequence listing 1.

11. A recombinant DNA molecule according to claim 9, further comprising one or more structural genes wherein the said structural gene(s) is (are) genetically linked with the structural gene coding for the S-locus

EP 0 436 467 A2

specific glycoprotein.

**12.** A recombinant DNA molecule according to claim 11 wherein the said structural gene is capable of imparting herbicide tolerance to the recipient plant.

**13.** A recombinant DNA molecule according to claim 12 wherein the gene for herbicide tolerance is capable of imparting herbicide tolerance to one or more herbicides selected from the group consisting of hygromycin, kanamycin, phosphinotricin, asulam, sulfonylureas, glyphosate, bromoxymil and imidazolinones.

**14.** A recombinant DNA transfer vector comprising :

(a) a structural gene which codes for the production of an S-locus specific glycoprotein from a self-incompatible plant ; and

(b) a transformation vector containing DNA capable of driving the expression of said structural gene when the construct is incorporated into a plant.

**15.** A recombinant DNA transfer vector comprising a recombinant DNA molecule according to any one of claims 9 to 13 incorporated into a transformation vector containing DNA capable of driving the expression of said structural gene when the construct is incorporated into a plant.

**16.** A self-incompatible plant and the progeny thereof comprising a structural gene which encodes for the production of an S-locus specific glycoprotein, wherein said plant is not ordinarily self-incompatible.

**17.** A self-incompatible plant and the progeny thereof comprising a structural gene which encodes for the production of an S-locus specific glycoprotein, wherein said plant does not ordinarily produce said S-locus specific glycoprotein.

**18.** A self-incompatible plant produced by the method of any one of claims 1 to 8.

**19.** A self-incompatible plant and the progeny thereof comprising a structural gene which encodes for the production of an S-locus specific glycoprotein, said structural gene being genetically linked with one or more further structural genes.

**20.** A self-incompatible plant according to claim 19 wherein the said further structural gene is capable of imparting herbicide tolerance, wherein said plant does not ordinarily produce said S-locus specific glycoprotein.

**21.** A self-incompatible plant and the progeny thereof according to claim 20 wherein the plant is tolerant to one or more herbicides selected from the group consisting of hygromycin, kanamycin, phosphinotricin, asulam, sulfonylureas, glyphosate, bromoxymil and imidazolinones.

**22.** A recombinant vector comprising a structural gene which codes for the production of an S-locus specific glycoprotein.

**23.** A recombinant vector according to claim 22 wherein the recombinant vector carries a gene coding for the production of an S-locus specific glycoprotein selected from the group consisting of the S13 SLSG, S14 SLSG, S22 SLSG glycoproteins of *Brassica oleracea* and the S8 SLSG glycoprotein of *Brassica campestris.*

**24.** A method of obtaining tissue specific expression of a gene in a plant comprising :

(a) isolating a tissue specific plant promoter from the reproductive tissue of a donor plant ;

(b) operably linking the tissue specific plant promoter with a structural gene such that the structural gene is specifically expressed only in the reproductive tissue of a recipient plant.

**25.** A method according to claim 24 wherein the tissue specific plant promoter causes the specific expression of the structural gene in the pistil of the recipient plant.

**26.** A method according to claim 24 wherein the tissue specific plant promoter causes the specific expression of the structural gene in the anther of the recipient plant.

**27.** A tissue specific plant promoter comprising a DNA sequence comprising a region of an S-locus specific gene that directs the expression of a linked structural gene in the reproductive tissue of a recipient plant.

**28.** A tissue specific plant promoter according to claim 27 further comprising a region of the SLG-13 gene that directs expression of a linked structural gene in the pistil of the recipient plant.

**29.** A tissue specific plant promoter according to Claim 28 comprising the region extending from approximately position -17 to approximately position -411 of the SLG-13 gene.

**30.** A tissue specific plant promoter according to Claim 29 comprising the region extending from approximately position -150 to approximately position -411 of the SLG-13 gene.

**31.** A recombinant DNA molecule according to any one of claims 9 to 13 comprising a tissue specific plant promoter comprising a region of an S-locus specific gene that directs the expression of a linked structural gene in the reproductive tissue of a recipient plant

**32.** A recombinant DNA transfer vector comprising :

(a) a tissue specific plant promoter comprising a DNA sequence comprising a region of an S-locus specific gene that directs the expression of a linked structural gene in the reproductive tissue of a recipient plant ;

(b) a structural gene which codes for the production of an S-locus specific glycoprotein from a self-incompatible plant ; and

c) a transformation vector containing DNA capable of driving the expression of said structural gene when

36

the construct is incorporated into a plant.

33. A method for obtaining a self-incompatible plant from a species which is not normally self-incompatible which comprises :

(a) isolating a tissue specific plant promoter from the reproductive tissue of a donor plant ;

(b) operably lining the tissue specific plant promoter with a DNA sequence comprising a region of a S-locus specific gene of sufficient length to express a S-locus glycoprotein substantially similar to that expressed in *Brassica* sp. ;

(c) fusing a reporter gene to the resulting linked promoter-gene construct ;

(d) inserting the resulting chimeric gene construction into the genome of a recipient plant which is not normally self-incompatible ;

(e) allowing the recipient plant to mature and assaying the flower parts of the mature plant for the expression of the S-locus glycoprotein and/or the reporter gene ; and

(f) selecting those plants which show expression of the S-locus glycoprotein and/or the reporter gene.

34. A method according to claim 33, wherein the reporter gene is a gene capable of imparting herbicide tolerance to the recipient plant.

35. A recombinant DNA molecule comprising the fusion of :

(a) a tissue specific plant promoter comprising a DNA sequence comprising a region of an S-locus specific gene that directs the expression of a linked structural gene in the reproductive tissue of a recipient plant ;

(b) a structural gene which codes for the production of an S-locus specific glycoprotein substantially similar in activity to a S-locus specific glycoprotein gene of *Brassica* sp. ; and

(c) one or more furter structural gene(s).

36. A recombinant DNA molecule according to claim 35, wherein the structural gene(s) of part (c) is a reporter gene the expression of which in a transgenic cell is indicative of the incorporation of the structural gene encoding a S-locus glycoprotein into the transgenic cell's genome.

37. A recombinant DNA molecule according to claim 35, wherein the structural gene(s) of part (c) is a gen capable of imparting herbicide tolerance.

38. A recombinant DNA transfer vector comprising a recombinant DNA molecule according to any one of claims 35 to 37 incorporated into a plant transformation vector.

39. A recombinant plant and the progeny thereof comprising multiple structural genes which encode for the production of an S-locus specific glycoprotein wherein said recombinant plant ordinarily comprises fewer structural genes encoding for the production of S-locus specific glycoproteins.

40. A recombinant plant according to claim 39 wherein at least one of the structural genes coding for the production of an S-locus specific glycoprotein in said recombinant plant is not ordinarily present in said recombinant plant.

41. A method for obtaining a male and/or female sterile plant which method comprises the following steps:

(a) isolating a tissue specific plant promoter from the reproductive tissue of a donor plant ;

(b) operably liking the tissue specific plant promoter with a DNA sequence that encodes for a polypeptide or protein taht is toxic to the recipient plant ;

(c) inserting the resulting chimeric gene into the genome of a recipient plant ;

(d) allowing the recipient plant to mature and assaying the flower parts of the mature plant for the expression of the toxin gene ; and

(e) selecting those plants which show expression of the toxin gene.

42. A recombinant DNA molecule comprising the fusion of :

(a) a tissue specific plant promoter comprising a DNA sequence comprising a region of an S-locus specific gene that directs the expression of a linked structural gene in the reproductive tissue of a recipient plant ;

(b) a structural gene which codes for the production of an polypeptide or a protein that is toxic to the recipient plant.

42. A female and/or male sterile plant and the asexually derived progeny thereof that still contains the trait of male and/or female sterility, comprising a structural gene which encodes for the production of a polypeptide or a protein that is toxic to the recipient plant, operably linked to a plant specific promoter that is capable of directing the expression of the associated toxin gene in the reproductive tissue, preferably in the anther and/or pistil, of a recipient plant.

43. A probe that specifically hybridizes to the 3' end of an S-locus structural gene.

44. A probe according to claim 43 wherein the S-locus specific structural gene is SLG-8, SLG-13, SLG-14, SLG-22, and SLG-2.

45. A probe according to claim 43 wherein the probe is specific to the region 2107 to 2216 in the S13 sequence.

46. A method for hybrid production of self-incompatible plants comprising :

(a) breeding a line of self-incompatible plants that comprises a structural gene which encodes for the production of an S-locus glycoprotein linked to a gene capable of imparting herbicide resistance ;

(b) breeding a line of self-fertile plants that is not resistant to herbicide ;

(c) interplanting the self-incompatible line and self-fertile line to produce hybrid plants ; and

(d) applying herbicide to which the self-incompatible plants are resistant to hybrid production fields after pollination and prior to harvest ;

whereby the surviving plants are all self-incompatible hybrid plants.

47. A method for hybrid production of self-incompatible hybrid plants comprising :

(a) breeding a line of self-incompatible plants that comprises a structural gene which encodes for the production of an S-locus glycoprotein linked to a gene capable of imparting herbicide resistance ;

(b) breeding a line of self-fertile plants that is not resistant to herbicide ;

(c) planting the line of self-incompatible plants and the line of self-fertile plants in separate strips ; and

(d) applying herbicide to herbicide self-fertile plants before harvest ;

whereby the surviving plants are all self-incompatible hybrid plants.

48. A method for the maintenance of an inbred line of plants in heterozygous form comprising :

(a) crossing self-incompatible, herbicide resistant plants with self-fertile, herbicide sensitive plants ;

(b) germinating seeds from the cross ; and

(c) applying herbicide to the germinated seeds ;

whereby the surviving plants are all heterozygous self-incompatible plants.

49. A method for hybrid production of self-incompatible hybrid plants comprising :

(a) breeding a first line of self-incompatible plants that comprises a first structural gene which encodes for the production of an S-locus glycoprotein linked to a gene capable of imparting herbicide resistance ;

(b) breeding a second line of self-incompatible plants that comprises a second structural gene which encodes for the production of an S-locus glycoprotein linked to a gene capable of imparting herbicide resistance ; and

(c) interplanting the first and second lines of self-incompatible plants to produce hybrid plants.

50. A method for the maintenance of an inbred line of plants in heterozygous form comprising :

(a) crossing a first line of self-incompatible, herbicide resistant plants with a second line of self-incompatible, herbicide resistant plants, said first and second lines of self-incompatible plants comprising different structural genes which encode for the production of different S-locus glycoproteins and are linked to a gene capable of imparting herbicide resistance ;

(b) germinating seeds from the cross ; and

(c) applying herbicide to the germinated seeds ;

whereby the surviving plants are all heterozygous self-incompatible plants.

## Claims for the following Contracting State : SP

1. A method of imparting self-incompatibility to recipient plants comprising :

   (a) isolating a structural gene which encodes for the production of an S-locus specific glycoprotein from a self-incompatible donor plant ; and

   (b) integrating said structural gene into the genome of a recipient plant that does not ordinarily produce said S-locus specific glycoprotein, wherein said recipient plant is self-incompatible.

2. A method according to claim 1 wherein the donor plant is a self-incompatible strain of *Brassica.*

3. A method according to claim 1 wherein the structural gene is selected from the group consisting of the SLG-13, S-14, SLG-22 gene of *Brassica oleracea* and the SLG-8 gene of *Brassica campestris.*

4. A method according to claim 1 wherein the recipient plant is from a different family of plants from the self-incompatible donor plant.

5. A method according to claim 1 wherein said structural gene is stably integrated into the genome of the recipient plant using a strain of *Agrobacterium tumefaciens* cells as the means for integration.

6. A method according to claim 1 wherein the structural gene comprises the DNA sequence as given in sequence listing 1.

7. A method according to claim 1 wherein structural gene which encodes for the production of an S-locus specific glycoprotein from a self-incompatible donor plant is operably linked with one or more further structural genes.

8. A method according to claim 7 wherein the said further structural gene is a reporter gene the expression of which in a transgenic cell is indicative of the incorporation of the linked first structural gene into the trangenic cell's genome.

9. A method of producing a recombinant DNA molecule comprising
   (a) isolating a structural gene which codes for the production of an S-locus specific glycoprotein from a self-incompatible plant, selected from the group consisting of the SLG-13, SLG-14, SLG-22 genes of *Brassica oleracea* and the SLG-8 gene of *Brassica campestris* ;
   (b) ligating the said structural gene with plant expression signals.

10. A method according to claim 9 wherein the structural gene which codes for the production of an S-locus specific glycoprotein comprises the DNA sequence as given in sequence listing 1.

11. A method according to claim 9, further comprising one or more structural genes wherein the said structural gene(s) is (are) genetically linked with the first structural gene coding for the S-locus specific glycoprotein.

12. A method according to claim 11 wherein the said structural gene is capable of imparting herbicide tolerance to the recipient plant.

13. A method according to claim 12 wherein the gene for herbicide tolerance is capable of imparting herbicide tolerance to one or more herbicides selected from the group consisting of hygromycin, kanamycin, phosphinotricin, asulam, sulfonylureas, glyphosate, bromoxymil and imidazolinones.

14. A method of producing a recombinant DNA transfer vector comprising :
   (a) isolating a structural gene which codes for the production of an S-locus specific glycoprotein from a self-incompatible plant ; and
   (b) splicing the said structural gene into a plant transformation vector containing DNA capable of driving the expression of said structural gene when the construct is incorporated into a plant.

15. A method of producing a self-incompatible plant and the progeny thereof comprising transforming a structural gene which encodes for the production of an S-locus specific glycoprotein into a recipient plant, wherein said plant is not ordinarily self-incompatible.

16. A method according to claim 15, wherein the said plant does not ordinarily produce said S-locus specific glycoprotein.

17. A method of producing a self-incompatible plant and the progeny thereof comprising transforming a recipient plant, which is not ordinarily self-incompatible with a genetic construct comprising a structural gene encoding for the production of an S-locus specific glycoprotein, which is genetically linked with one or more further structural genes.

18. A method according to claim 17 wherein the said further structural gene is capable of imparting herbicide tolerance.

19. A method according to claim 17, wherein said recipient plant does not ordinarily produce said S-locus specific glycoprotein.

20. A method according to claim 18 wherein the plant is tolerant to one or more herbicides selected from the group consisting of hygromycin, kanamycin, phosphinotricin, asulam, sulfonylureas, glyphosate, bromoxymil and imidazolinones.

21. A method of obtaining tissue specific expression of a gene in a plant comprising :
   (a) isolating a tissue specific plant promoter from the reproductive tissue of a donor plant ;
   (b) operably linking the tissue specific plant promoter with a structural gene such that the structural gene is specifically expressed only in the reproductive tissue of a recipient plant.

39

22. A method according to claim 21 wherein the tissue specific plant promoter causes the specific expression of the structural gene in the pistil of the recipient plant.

23. A method according to claim 21 wherein the tissue specific plant promoter causes the specific expression of the structural gene in the anther of the recipient plant.

24. A method according to claim 21 wherein the tissue specific plant promoter is obtainable from a region of an S-locus specific gene that directs the expression of a linked structural gene in the reproductive tissue of a recipient plant.

25. A method according to claim 24 wherein the tissue specific plant promoter comprises the region extending from approximately position -17 to approximately position -411 of the SLG-13 gene.

26. A method according to claim 25 wherein the tissue specific plant promoter comprises the region extending from approximately position -150 to approximately position -411 of the SLG-13 gene.

27. A method according to any one of claims 9 to 13 wherein the plant expression signals comprise a tissue specific plant promoter comprising a region of an S-locus specific gene that directs the expression of a linked structural gene in the reproductive tissue of a recipient plant

28. A method of producing a plant transformation vector comprising :
    (a) isolating a tissue specific plant promoter comprising a DNA sequence comprising a region of an S-locus specific gene that directs the expression of a linked structural gene in the reproductive tissue of a recipient plant ;
    (b) ligating a structural gene which codes for the production of an S-locus specific glycoprotein from a self-incompatible plant with the said tissue specific plant promoter, and
    (c) splicing the resulting chimeric construct into a plant transformation vector.

29. A method for obtaining a self-incompatible plant from a species which is not normally self-incompatible which comprises :
    (a) isolating a tissue specific plant promoter from the reproductive tissue of a donor plant ;
    (b) operably linking the tissue specific plant promoter with a DNA sequence comprising a region of a S-locus specific gene of sufficient length to express a S-locus glycoprotein substantially similar to that expressed in Brassica sp. ;
    (c) fusing a reporter gene to the resulting linked promoter-gene construct ;
    (d) inserting the resulting chimeric gene construction into the genome of a recipient plant which is not normally self-incompatible ;
    (e) allowing the recipient plant to mature and assaying the flower parts of the mature plant for the expression of the S-locus glycoprotein and/or the reporter gene ; and
    (f) selecting those plants which show expression of the S-locus glycoprotein and/or the reporter gene.

30. A method according to claim 29, wherein the reporter gene is a gene capable of imparting herbicide tolerance to the recipient plant.

31. A method of producing a recombinant DNA molecule comprising fusing
    (a) a tissue specific plant promoter comprising a DNA sequence comprising a region of an S-locus specific gene that directs the expression of a linked structural gene in the reproductive tissue of a recipient plant ;
    (b) a structural gene which codes for the production of an S-locus specific glycoprotein substantially similar in activity to a S-locus specific glycoprotein gene of Brassica sp. ; and
    (c) one or more further structural gene(s).

32. A method according to claim 31, wherein the structural gene(s) of part (c) is a reporter gene the expression of which in a transgenic cell is indicative of the incorporation of the structural gene encoding a S-locus glycoprotein into the transgenic cell's genome.

33. A method according to claim 31, wherein the structural gene(s) of part (c) is a gen capable of imparting herbicide tolerance.

**34.** A method of producing a recombinant transfer vector comprising splicing a recombinant DNA molecule according to any one of claims 31 to 33 into a plant transformation vector.

**35.** A method for obtaining a male and/or female sterile plant which method comprises the following steps :
(a) isolating a tissue specific plant promoter from the reproductive tissue of a donor plant ;
(b) operably liking the tissue specific plant promoter with a DNA sequence that encodes for a toxin polypeptide or protein ;
(c) inserting the resulting chimeric gene into the genome of a recipient plant ;
(d) allowing the recipient plant to mature and assaying the flower parts of the mature plant for the expression of the toxin gene ; and
(e) selecting those plants which show expression of the toxin gene.

**36.** A method of producing a recombinant DNA molecule comprising fusing
(a) a tissue specific plant promoter comprising a DNA sequence comprising a region of an S-locus specific gene that directs the expression of a linked structural gene in the reproductive tissue of a recipient plant ;
(b) a structural gene which codes for the production of an polypeptide or a protein that is toxic to the recipient plant.

**37.** A method of producing a female and/or male sterile plant and the asexually derived progeny thereof that still contains the trait of male and/or female sterility comprising transforming a recipient plant with a plant transformation vector comprising a structural gene which encodes for the production of a polypeptide or a protein that is toxic to the recipient plant, operably linked to a plant specific promoter that is capable of directing the expression of the associated toxin gene in the reproductive tissue.

**38.** A method for hybrid production of self-incompatible plants comprising :
(a) breeding a line of self-incompatible plants that comprises a structural gene which encodes for the production of an S-locus glycoprotein linked to a gene capable of imparting herbicide resistance ;
(b) breeding a line of self-fertile plants that is not resistant to herbicide ;
(c) interplanting the self-incompatible line and self-fertile line to produce hybrid plants ; and
(d) applying herbicide to which the self-incompatible plants are resistant to hybrid production fields after pollination and prior to harvest ;
whereby the surviving plants are all self-incompatible hybrid plants.

**39.** A method for hybrid production of self-incompatible hybrid plants comprising :
(a) breeding a line of self-incompatible plants that comprises a structural gene which encodes for the production of an S-locus glycoprotein linked to a gene capable of imparting herbicide resistance ;
(b) breeding a line of self-fertile plants that is not resistant to herbicide ;
(c) planting the line of self-incompatible plants and the line of self-fertile plants in separate strips ; and
(d) applying herbicide to herbicide self-fertile plants before harvest ;
whereby the surviving plants are all self-incompatible hybrid plants.

**40.** A method for the maintenance of an inbred line of plants in heterozygous form comprising :
(a) crossing self-incompatible, herbicide resistant plants with self-fertile, herbicide sensitive plants ;
(b) germinating seeds from the cross ; and
(c) applying herbicide to the germinated seeds ;
whereby the surviving plants are all heterozygous self-incompatible plants.

**41.** A method for hybrid production of self-incompatible hybrid plants comprising :
(a) breeding a first line of self-incompatible plants that comprises a first structural gene which encodes for the production of an S-locus glycoprotein linked to a gene capable of imparting herbicide resistance;
(b) breeding a second line of self-incompatible plants that comprises a second structural gene which encodes for the production of an S-locus glycoprotein linked to a gene capable of imparting herbicide resistance ; and
(c) interplanting the first and second lines of self-incompatible plants to produce hybrid plants.

**42.** A method for the maintenance of an inbred line of plants in heterozygous form comprising :
(a) crossing a first line of self-incompatible, herbicide resistant plants with a second line of self-incompatible, herbicide resistant plants, said first and second lines of self-incompatible plants comprising different structural genes which encode for the production of different S-locus glycoproteins and are linked to a gene capable of imparting herbicide resistance ;

(b) germinating seeds from the cross ; and

(c) applying herbicide to the germinated seeds ;

whereby the surviving plants are all heterozygous self-incompatible plants.

FIG. 1

SLG-8

SLG-14

SLG-22

SLG-2

FIG. 2

**FIG. 3**

| DAYS BEFORE ANTHESIS | MICROSPORE AND POLLEN STAGES | GUS ACTIVITY IN | |
|---|---|---|---|
| | | ANTHER | STIGMA |
| 9 | PRE-MEIOSIS | | |
| 8 | | | |
| 7 | TETRAD | | |
| 6 | UNI-NUCLEATE | | |
| 5 | | | |
| 4 | BI-NUCLEATE | | |
| 3 | | | |
| 2 | TRI-NUCLEATE | | |
| 1 | | | |
| 0 | | | |

# FIG. 4